(19) Europäisches Patentamt
European Patent Office
Office européen des brevets

(11) **EP 3 088 897 B1**

(12) **EUROPEAN PATENT SPECIFICATION**

(45) Date of publication and mention
of the grant of the patent:
**04.11.2020 Bulletin 2020/45**

(51) Int Cl.:
***G01N 33/68*** (2006.01)

(21) Application number: **14867880.8**

(22) Date of filing: **04.12.2014**

(86) International application number:
**PCT/JP2014/082061**

(87) International publication number:
**WO 2015/083765 (11.06.2015 Gazette 2015/23)**

(54) **METHOD FOR PREDICTING THERAPEUTIC EFFECT OF BIOLOGICAL PREPARATION ON RHEUMATOID ARTHRITIS**

VERFAHREN ZUR VORHERSAGE DER WIRKSAMKEIT EINES BIOLOGISCHEN PRÄPARATS BEI RHEUMATOIDER ARTHRITIS

PROCÉDÉ PERMETTANT DE PRÉVOIR L'EFFET THÉRAPEUTIQUE D'UNE PRÉPARATION BIOLOGIQUE SUR L'ARTHRITE RHUMATOÏDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.12.2013 JP 2013251152**

(43) Date of publication of application:
**02.11.2016 Bulletin 2016/44**

(73) Proprietor: **Yoshizaki, Kazuyuki**
**Osaka 567-0047 (JP)**

(72) Inventors:
• **YOSHIZAKI, Kazuyuki**
  **Ibaraki-shi**
  **Osaka 567-0047 (JP)**
• **UNO, Kazuko**
  **Kyoto-shi**
  **Kyoto 606-8225 (JP)**
• **IWAHASHI, Mitsuhiro**
  **Higashihiroshima-shi**
  **Hiroshima 739-0002 (JP)**
• **YAGI, Katsumi**
  **Kyoto-shi**
  **Kyoto 606-8225 (JP)**

(74) Representative: **Uexküll & Stolberg**
**Partnerschaft von**
**Patent- und Rechtsanwälten mbB**
**Beselerstraße 4**
**22607 Hamburg (DE)**

(56) References cited:
**WO-A2-2007/038501**

• **PETER OELZNER ET AL: "The balance between soluble receptors regulating IL-6 trans-signaling is predictive for the RANKL/osteoprotegerin ratio in postmenopausal women with rheumatoid arthritis", RHEUMATOLOGY INTERNATIONAL ; CLINICAL AND EXPERIMENTAL INVESTIGATIONS, SPRINGER, BERLIN, DE, vol. 32, no. 1, 7 September 2010 (2010-09-07), pages 199-206, XP019999332, ISSN: 1437-160X, DOI: 10.1007/S00296-010-1606-Z**
• **S. FABRE ET AL: "Protein biochip array technology for cytokine profiling predicts etanercept responsiveness in rheumatoid arthritis", CLINICAL & EXPERIMENTAL IMMUNOLOGY, vol. 153, no. 2, 1 August 2008 (2008-08-01), pages 188-195, XP055075047, ISSN: 0009-9104, DOI: 10.1111/j.1365-2249.2008.03691.x**
• **UNO KAZUKO ET AL: "Pretreatment Prediction of Individual Rheumatoid Arthritis Patients' Response to Anti-Cytokine Therapy Using Serum Cytokine/Chemokine/Soluble Receptor Biomarkers", PLOS ONE, vol. 10, no. 7, E0132055, July 2015 (2015-07), XP002770506,**
• **SHUSAKU NAKASHIMA ET AL.: 'Tocilizumab no Chiryo Hannosei o Shimesu Biomarker no Kento' JAPAN COLLEGE OF RHEUMATOLOGY SOKAI GAKUJUTSU SHUKAI KOKUSAI SYMPOSIUM PROGRAM SHOROKUSHU vol. 57 TH-22, 19 March 2013, page 419, XP008183936**

EP 3 088 897 B1

- **HIROSHI SAWANO: 'MMP-3 is a Predictive Factor for Clinical Disease Activity Index Remission in Tocilizumab Treatment of Rheumatoid Arthritis' JAPANESE JOURNAL OF JOINT DISEASES vol. 31, no. 2, 01 January 2012, pages 115 - 119, XP055348111 DOI: 10.11551/JSJD.31.115**
- **RYO TAKAHASHI: 'Seibutsugakuteki Seizai Koka Yosoku Inshi to shite no Ko CCP Kotai' RHEUMATOLOGY vol. 48, no. 2, 28 August 2012, pages 225 - 230, XP008183955**
- **KEISUKE IZUMI: 'Bunshi Hyotekiyaku Chiryo no Yogo to Chiryo Seiseki Hyoka Rheumatism Hyotekiyaku no Yukosei Yosoku Inshi' JAPANESE JOURNAL OF CLINICAL MEDICINE vol. 70, 20 November 2012, pages 658 - 663, XP008183770**
- **Shusaku Nakashima ET AL: "W72-2 Analysis of predictive biomarker which reflects the efficacy of Tocilizumab treatment for RA patient", Modern Rheumatology, vol. 23, no. Suppl. 2013, 19 March 2013 (2013-03-19), - 20 April 2013 (2013-04-20), page S154, XP055356197, JP ISSN: 1439-7595**

**Description**

[Technical Field]

The present invention relates to a method of predicting and determining a therapeutic effect of a biological formulation on a rheumatoid arthritis patient. More specifically, the present invention relates to a method of predicting and determining a therapeutic effect, such as the level of improvement in a symptom or the possibility of remission, prior to the administration of a biological formulation to a rheumatoid arthritis patient. Furthermore, the present invention relates to the use of a diagnostic agent for predicting and determining a therapeutic effect due to a biological formulation on a rheumatoid arthritis patient.

[Background Art]

Rheumatoid arthritis is a systemic inflammatory disease, which is predominantly a lesion in the articular synovial membrane. It is estimated that approximately 700,000 people suffer from rheumatoid arthritis in Japan. Many biological formulations that target inflammatory cytokines have been developed for rheumatoid arthritis therapy. In recent years, anti-TNF-a agents or anti-IL-6 agents, which inhibit TNF-$\alpha$ or IL-6 action, have been used in clinical practices.

Conventionally, biological formulations targeting an inflammatory cytokine, such as tocilizumab, etanercept, adalimumab, or infliximab, have been used in rheumatoid arthritis therapy. Tocilizumab is a humanized IL-6 receptor antibody, which is an agent that causes rheumatoid arthritis to subside by the action of binding to a membrane-binding IL-6 receptor and a soluble IL-6 receptor to suppress IL-6 signaling. Further, etanercept is a fully human soluble TNF/LT$\alpha$ receptor formulation consisting of a subunit dimer of an extracellular domain of a human tumor necrosis factor II receptor and an Fc region of a human IgG1. Etanercept is an agent that binds to both TNF$\alpha$/$\beta$ to inhibit signaling to a TNF receptor to cause rheumatoid arthritis to subside. Adalimumab and infliximab are human and chimeric TNF-$\alpha$ antibodies, which are agents that cause rheumatoid arthritis to subside by specifically binding to excessively produced TNF-$\alpha$ and inhibiting the binding of TNF-$\alpha$ to a TNF-$\alpha$ receptor.

For such biological formulations, a certain level of effectiveness in rheumatoid arthritis therapy is verified, while such formulations have disadvantages such as the formulations being expensive and time-intensive for determining a therapeutic effect. In addition, there are certain percentages of cases with no effect, and expression of side effects, such as an infectious disease or an interstitial pneumonia, has been observed in some cases. For this reason, cases where the biological formulation is usable are limited. Thus, if the effectiveness of a biological formulation targeting an inflammatory cytokine can be estimated in advance for each rheumatoid arthritis patient, this would be a boon to rheumatoid arthritis patients and provide contribution to medical business.

Markers for predicting the effectiveness of a biological formulation targeting an inflammatory cytokine on a rheumatoid arthritis patient have been intensively investigated. For example, a method of using a microDNA chip, a method of using a CRP value at baseline as an indicator, a method of using blood soluble ICAMI concentration and CXCL13 concentration as indicators, a method of using leukocyte ADAMT5 gene expression amount as an indicator (Non Patent Literature 4), a method of comprehensively analyzing genetic polymorphisms (Patent Literatures 1 and 2), a method of analyzing a genetic mutation of an IL-6 receptor (Patent Literature 3) and the like have been reported as a method of predicting the therapeutic effectiveness of tocilizumab on rheumatoid arthritis. Further, a method of analyzing the IL10RB gene, the IRF5 gene, and polymorphisms of the IRF5 gene (Patent Literature 4) has been reported as a method of predicting the therapeutic effectiveness of infliximab on rheumatoid arthritis. Furthermore, a method of comprehensively analyzing genetic polymorphisms (Patent literature 5) has been reported as a method of predicting the therapeutic effectiveness of an anti-TNF-a agent such as etanercept, adalimumab, or infliximab on rheumatoid arthritis.

However, conventional methods of determining a therapeutic effect on rheumatoid arthritis have disadvantages such as: genetic analysis or the like is required, in addition to the operation being complicated; analysis is time and cost-intensive; there is little versatility; proper diagnosis rate is low; and the like. Furthermore, conventional approaches cannot accurately determine whether rheumatoid arthritis can be in full remission prior to the administration of a biological formulation. Thus, conventional approaches have a problem in that an appropriate therapeutic plan which takes into consideration the therapeutic effect thereof cannot be established prior to administration of a biological formulation.

Such background conventional techniques elicit a desire for the establishment of a technique for predicting a therapeutic effect of biological formulation administration to a rheumatoid arthritis patient, which is simple and cost-efficient, highly versatile and highly accurate.

[Citation List]

[Patent Literature]

[PTL 1] International Publication No. WO 2011/128096
[PTL 2] Japanese Laid-Open Publication No. 2011-182780
[PTL 3] International Publication No. WO 2012/41332
[PTL 4] Japanese Laid-Open Publication No. 2009-225713
[PTL 5] Japanese Laid-Open Publication No. 2010-088432

[Summary of Invention]

[Solution to Problem]

The objective of the present invention is to provide a method of predicting and determining a therapeutic effect (level of improvement in a symptom or possibility of remission) prior to administration of a biological formulation, which is simple and cost-effective, highly versatile and highly accurate. Further objective of the present invention is to provide the use of a diagnostic agent for carrying out the above-described method.

The inventors have analyzed the prognostic state of a rheumatoid arthritis patient administered with the biological formulation and the concentrations of cytokines , chemokines and soluble receptors thereof in a serum of the patient prior to administration of the biological formulation in order to solve the problem to discover that a therapeutic effect on a rheumatoid arthritis patient (e.g., level of improvement in a symptom or possibility of remission) can be predicted and determined in a simple and cost-effective manner at any facility with high accuracy, prior to administering a biological formulation targeting an inflammatory cytokine by utilizing the serum concentration of one or more types selected from the group consisting of sgp130, IP-10, sTNFRI, sTNFRII, GM-CSF, IL-1$\beta$, IL-2, IL-5, IL-6, IL-7, IL-8, IL-9, IL-10, IL-12, IL-13, IL-15, Eotaxin, VEGF, MCP-1, TNF-$\alpha$, IFN-$\gamma$, FGFbasic, PDGF-bb, sIL-6R, and MIP-1$\alpha$. The invention is defined by the appended claims.

More specifically, the inventors have obtained the following knowledge.

(1-1) It was discovered through simple linear regression analysis that when therapy is applied by administering tocilizumab to a rheumatism patient who has not received anti-cytokine therapy (administration of infliximab, etanercept, adalimumab, tocilizumab or the like) in the past (may also be referred to as a "naive patient" hereinafter), the level of improvement in the DAS-28 value (DAS-28 value prior to therapy - DAS-28 value after 16 weeks of therapy) is significantly correlated with the log values of serum concentrations of IL-7, IL-8, IL-12, IL-13, IP-10, and VEGF prior to the administration of tocilizumab to the patient.

(1-2) It was discovered through simple linear regression analysis that when therapy is applied by administering tocilizumab to a rheumatism patient who has received anti-cytokine therapy in the past (also referred to as a "switch patient" hereinafter), the level of improvement in the DAS-28 value is significantly correlated with the log values of serum concentrations of IL-1$\beta$, IL-5, IL-6, IL-7, IL-10, IL-12, IL-13, IL-15, FGFbasic, GM-CSF, IFN-$\gamma$, TNF-$\alpha$, and VEGF prior to the administration of tocilizumab to the patient.

(1-3) It was discovered through simple linear regression analysis that when therapy is applied by administering etanercept to a naive patient, the level of improvement in DAS-28 value is significantly correlated with the log values of serum concentrations of IL-6 and IP-10 prior to the administration of etanercept to the patient.

(1-4) It was discovered through multiple linear regression analysis that when therapy is applied by administering tocilizumab to a naive patient, the level of improvement in DAS-28 value is significantly correlated with a combination of log values of serum concentrations of IL-1$\beta$, IL-7, TNF-$\alpha$, and sIL-6R prior to the administration of tocilizumab to the patient.

(1-5) It was discovered through multiple linear regression analysis that when therapy is applied by administering etanercept to a naive patient, the level of improvement in DAS-28 value is significantly correlated with a combination of log values of serum concentrations of IL-2, IL-15, sIL-6R, and sTNFRI prior to the administration of etanercept to the patient.

(2-1) It was discovered through simple linear regression analysis that when therapy is applied by administering tocilizumab to a naive patient, the DAS-28 value after 16 weeks of therapy is significantly correlated with a serum concentration of sgp130 prior to the administration of tocilizumab to the patient.

(2-2) It was discovered through simple linear regression analysis that when therapy is applied by administering tocilizumab to a switch patient, the DAS-28 value after 16 weeks of therapy is significantly correlated with the log values of serum concentrations of IL-1β, IL-2, IL-5, IL-15, GM-CSF, IFN-γ, and TNF-α and a serum concentration of sgp130 prior to the administration of tocilizumab to the patient.

(2-3) It was discovered through simple linear regression analysis that when therapy is applied by administering etanercept to a naive patient, the DAS-28 value after 16 weeks of therapy is significantly correlated with the log value of a serum concentration of IL-9 prior to the administration of etanercept to the patient.

(2-4) It was discovered through multiple linear regression analysis that when therapy is applied by administering tocilizumab to a naive patient, the DAS-28 value after 16 weeks of therapy is significantly correlated with a combination of log values of serum concentrations of IL-8, Eotaxin, IP-10, sTNRFI, sTNFRII, IL-6 and VEGF and a serum concentration of sgp130 prior to the administration of tocilizumab to the patient.

(2-5) It was discovered through multiple linear regression analysis that when therapy is applied by administering tocilizumab to a naive patient, the DAS-28 value after 16 weeks of therapy is significantly correlated with a combination of log values of serum concentrations of IL-8, Eotaxin, IP-10, sTNFRI, sTNRFII, and IL-6 and a serum concentration of sgpl30 prior to the administration of tocilizumab to the patient.

(2-6) It was discovered through multiple linear regression analysis that when therapy is applied by administering tocilizumab to a switch patient, the DAS-28 value after 16 weeks of therapy is significantly correlated with a combination of log values of serum concentrations of IP-10 and GM-CSF and a serum concentration of sgp130 prior to the administration of tocilizumab to the patient.

(2-7) It was discovered through multiple linear regression analysis that when therapy is applied by administering etanercept to a naive patient, the DAS-28 value after 16 weeks of therapy is significantly correlated with a combination of log values of serum concentrations of IL-6 and IL-13 and the DAS-28 value prior to the administration of etanercept.

(2-8) It was discovered through multiple linear regression analysis that when therapy is applied by administering etanercept to a naive patient, the DAS-28 value after 16 weeks of therapy is significantly correlated with a combination of log values of serum concentrations of IL-9, TNF-α, and VEGF prior to the administration of etanercept.

(3-1) It was discovered through multiple logistic regression analysis that the possibility of remission, when therapy is applied by administering tocilizumab to a naive patient can be predicted and determined by combining a serum concentration of sgp130, a log value of a serum concentration of IP-10, a log value of a serum concentration of sTNFRII, and a log value of a serum concentration of IL-6, IL-7, MCP-1, or IL-1β prior to the administration of tocilizumab.

(3-2) It was discovered through multiple logistic regression analysis that the possibility of remission, when therapy is applied by administering tocilizumab to a switch patient, can be predicted and determined by combining a serum concentration of sgp130, a log value of a serum concentration of IP-10, a log value of a serum concentration of sTNFRII, and a log value of a serum concentration of IL-6 or IL-1β prior to the administration of tocilizumab.

(3-3) It was discovered through multiple logistic regression analysis that the possibility of remission, when therapy is applied by administering etanercept to a naive patient, can be predicted and determined by combining the DAS-28 value and log values of serum concentrations of VEGF and PDGF-bb prior to the administration of etanercept.

(3-4) It was discovered through multiple logistic regression analysis that the possibility of remission, when therapy is applied by administering etanercept to a naive patient, can be predicted and determined by combining the DAS-28 value and log values of serum concentrations of MIP-1α and PDGF-bb prior to the administration of etanercept.

(3-5) It was discovered through multiple linear regression analysis that the possibility of remission, when therapy is applied by administering etanercept to a naive patient, can be predicted and determined by combining log values of serum concentrations of IL-9 and TNF-α prior to the administration of etanercept.

The present invention was completed by additional repeated examinations based on such knowledge. Specifically, the present invention provides a method of predicting and determining a therapeutic effect of a biological formulation targeting an inflammatory cytokine on a rheumatoid arthritis patient, as defined in claim 1

Item 2. The method of item 1 of predicting and determining a possibility of remission with tocilizumab.

Item 3 The method of determining of item 2, wherein
a patient to be administered with tocilizumab is a rheumatoid arthritis patient who has not received anti-cytokine therapy in the past, and
the determination marker is a combination of (i) sgp130, (ii) IP-10, (iii) sTNFRII, and (iv) IL-6, IL-7, MCP-1 or IL-1β.

Item 4 The method of determining of item 2, wherein a patient to be administered with tocilizumab is a rheumatoid arthritis patient who has received anti-cytokine therapy in the past, and
the determination marker is a combination of (i) sgp130, (ii) IP-10, (iii) sTNFRII, and (iv) IL-6 or IL-1β.

Item 5. The method of determining of item 1, wherein

the method is a method of predicting and determining a disease activity indicator after therapy with tocilizumab in a rheumatism patient who has not received anti-cytokine therapy in the past, and
the determination marker is at least one type selected from the group consisting of sgp130, IL-8, Eotaxin, IP-10, sTNFRI, sTNFRII, IL-6, and VEGF.

Item 6. The method of determining of item 5, wherein the determination marker is a combination of sgp130, IP-10, and GM-CSF.

Item 7. A method of selecting a more effective biological formulation for therapy in a rheumatism patient who has not received anti-cytokine therapy in the past from among biological formulations consisting of tocilizumab and as defined in claim 7.

Item 8. A method of selecting a more effective biological formulation for therapy in a rheumatism patient who has not received anti-cytokine therapy in the past from among biological formulations consisting of tocilizumab and etanercept as defined in claim 8.

Item 9. Use of a diagnostic agent for predicting and determining a therapeutic effect due to a biological formulation targeting an inflammatory cytokine on a rheumatoid arthritis patient, as defined in claim 9.

[Advantageous Effects of Invention]

Accordingly to the present invention, a therapeutic effect on a rheumatoid arthritis patient can be accurately estimated, and whether rheumatoid arthritis would enter a state of complete remission due to a biological formulation can be determined with high precision, prior to the administration of the biological formulation targeting an inflammatory cytokine. Furthermore, according to the present invention, a level of improvement in a symptom for a rheumatoid arthritis patient can be accurately determined prior to the administration of the biological formulation, thus allowing the establishment of a suitable treatment plan, which takes into consideration the therapeutic effect of the biological formulation. Further, according to the present invention, it is possible to predict which biological formulation is the most effective when administered for a rheumatoid arthritis patient prior to therapy. Thus, the most effective treatment plan can be established for each patient by selecting the optimal biological formulation for each patient.

In this manner, a rheumatoid arthritis patient for whom administration of a biological formulation is effective can be identified by utilizing the present invention. Thus, for patients, the present invention is beneficial in terms of medical cost containment, sense of security from the prediction of a therapeutic effect and the like. For physicians, the present invention enables the establishment of a suitable treatment plan based on an accurate prediction of effectiveness of a biological formulation.

Furthermore, the present invention does not require complex and time-consuming genetic analysis which lacks versatility. In addition, the present invention uses the concentration of a specific cytokine, chemokine, and/or soluble receptor in a serum as an indicator. Thus, the present invention can estimate in advance the effectiveness of a biological formulation targeting an inflammatory cytokine for each patient in a simple and cost-effective manner by using an existing method of measurement.

[Brief Description of Drawings]

[Figure 1] Figure 1 is a diagram showing trial profiles of tocilizumab therapy patients and etanercept therapy patients.
[Figure 2] Figures 2-1 to 2-4 are diagrams showing clinical baseline individual group statistics for healthy individuals and rheumatoid arthritis patients with respect to serum concentrations of cytokines/chemokines/soluble receptors.
[Figure 3] Figure 3 is a diagram showing the relationship between DAS-28 values prior to therapy and DAS-28 values after 16 weeks of therapy in tocilizumab therapy patients and etanercept therapy patients.
[Figure 4] Figure 4 is a diagram showing the relationship between DAS-28 values prior to therapy (PreDAS-28 score) and values obtained from subtracting a DAS-28 value after 16 weeks from a DAS-28 value prior to therapy (PreDAS-28score - 16W DAS-28 score) in naive patients who received tocilizumab therapy.
[Figure 5] Figure 5 is a diagram showing results of comparing predicted DAS-28 values after 16 weeks of therapy calculated from regression equation (4) prior to therapy and actual DAS-28 values after 16 weeks of therapy subjecting naive patients who received tocilizumab therapy.
[Figure 6] Figure 6 is a diagram showing results of comparing predicted DAS-28 values after 16 weeks of therapy calculated from regression equation (5) prior to therapy and actual DAS-28 values after 16 weeks of therapy subjecting switch patients who received tocilizumab therapy.

[Figure 7] Figure **7** is a diagram showing results of comparing predicted DAS-28 values after 16 weeks of therapy calculated from regression equation (7) prior to therapy and actual DAS-28 values after 16 weeks of therapy subjecting naive patients who received etanercept therapy.

[Figure 8] Figure **8** is a diagram showing the relationship between actual values of DAS-28 after 16 weeks of etanercept therapy and predicted DAS-28 values after 16 weeks of therapy estimated by assuming a patient has received tocilizumab therapy in naive patients who received etanercept therapy.

[Figure 9] Figure **9** is a diagram showing results of analyzing the relationship between serum sgp130 concentrations and DAS-28 values prior to therapy for patients in remission and non-remission.

[Description of Embodiments]

## 1. Determining method

The present application describes a method of determining a therapeutic efficacy of a biological formulation targeting an inflammatory cytokine on a rheumatoid arthritis patient, characterized in comprising the step of measuring a concentration of one or more types selected from the group consisting of sgp130, IP-10, sTNFRI, sTNFRII, GM-CSF, IL-1$\beta$, IL-2, IL-5, IL-6, IL-7, IL-8, IL-9, IL-10, IL-12, IL-13, IL-15, Eotaxin, VEGF, MCP-1, TNF-$\alpha$, IFN-$\gamma$, FGFbasic, PDGF-bb, sIL-6R, and MIP-1$\alpha$ in a serum collected from the rheumatoid arthritis patient prior to the administration of the biological formulation. Hereinafter, the determining method of the present invention is discussed in detail.

### Biological formulation subjected to determination

The determining method of the present invention is a method of predicting and determining a therapeutic effect of a biological formulation targeting an inflammatory cytokine on a rheumatoid arthritis patient.

A biological formulation targeting an inflammatory cytokine is not particularly limited as long as it is a biological formulation used in rheumatoid arthritis therapy. A therapeutic effect can be predicted and determined in accordance with the type of biological formulation to be used in the determining method of the present invention. Examples of a biological formulation targeting an inflammatory cytokine include anti-IL-6 agents, anti-TNF-$\alpha$ agents. Specific examples of anti-IL-6 agent include humanized anti-IL-6 receptor antibodies, anti-TNF-a antibodies, human soluble TNF/LT$\alpha$ receptors consisting of an Fc region of human IgG1 and a subunit dimer of an extracellular domain of a human tumor necrosis factor receptor II, and the like. More specific examples of the humanized anti-IL-6 receptor antibodies include tocilizumab. Further, examples of the human soluble TNF/LT$\alpha$ receptors more specifically include etanercept. Further, examples of the anti-TNF-a antibodies more specifically include adalimumab and infliximab.

Examples of optimal biological formulations thereamong which are applied in the determining method of the present invention include humanized anti-IL-6 receptor antibodies and humanized soluble TNF/LT$\alpha$ receptors, and still preferably tocilizumab and etanercept.

### Patients subjected to determination

The determining method of the present invention determines whether administration of a biological formulation is effective in a rheumatoid arthritis patient prior to administration of the biological formulation.

Further, target rheumatoid arthritis patients in the determining method of the present invention are not particularly limited, as long as it is prior to administration of the biological formulation. In addition, whether DMARDs such as methotrexate are administered, past dosing history of anti-cytokine therapy (administration of infliximab etanercept, adalimumab, tocilizumab or the like) are not relevant. A therapeutic effect due to a biological formulation can be predicted and determined by selecting a desired determination marker in accordance with the past dosing history of the biological formulation in the determining method of the present invention.

### Determination markers

The determining method of the present disclosure uses one or two or more types of determination markers selected from the group consisting of sgp130 (soluble gp130), IP-10 (interferon-inducible protein 10), sTNFRI (soluble receptors for tumor necrosis factor type I), sTNFRII (soluble receptors for tumor necrosis factor type II), GM-CSF (granulocyte macrophage colony-stimulating factor), IL-1$\beta$ (interleukin-1$\beta$), IL-2 (interleukin-2), IL-5 (interleukin-5), IL-6 (interleukin-6), IL-7 (interleukin-7), IL-8 (interleukin-8), IL-9 (interleukin-9), IL-10 (interleukin-10), IL-12 (interleukin-12), IL-13 (interleukin-13), IL-15 (interleukin-15), Eotaxin, VEGF (vascular endothelial growth factor), MCP-1 (monocyte chemotactic protein-1), TNF-$\alpha$ (tumor necrosis factor-$\alpha$), IFN-$\gamma$ (interferon-$\gamma$), FGFbasic (basic fibroblast growth factor), PDGF-bb (platelet-derived growth factor bb), sIL-6R (soluble receptors for interleukin-6), and MIP-1$\alpha$ (macrophage inflammatory protein-1$\alpha$) in the serum of the rheumatoid arthritis patient.

One type of the aforementioned specific cytokine, chemokine, and soluble receptor may be used alone as a determination marker in the determining method of the present invention. However, it is preferable to use two or more types from thereamong in combination as a determination marker, from the viewpoint of predicting and determining a therapeutic effect due to a biological formulation at a higher precision.

The determination marker is appropriately selected and used, depending on the therapeutic effect to be predicted and determined, type of biological formulation to be administered, past dosing history of biological formulation or the like. Specific optimal examples of determination marker are shown below for each therapeutic effect to be predicted and determined.

<Cases where level of improvement in symptom after therapy (level of improvement in value of disease activity indicator; value of disease activity indicator prior to therapy - value of disease activity indicator after therapy) is predicted and determined for biological formulation>

For a naive patient administered with tocilizumab (hereinafter, also referred to as an "tocilizumab therapy naive patient"), it is preferable to use at least one type selected from the group consisting of IL-7, IL-8, IL-12, IL-13, IP-10, VEGF, IL-1$\beta$, TNF-$\alpha$, and sIL-6R as a determination marker. It is more preferable to use IL-1$\beta$, IL-7, TNF-$\alpha$, and sIL-6R in combination as a determination marker.

For a switch patient administered with tocilizumab (hereinafter, also referred to as a "tocilizumab therapy switch patient"), it is preferable to use at least one type selected from the group consisting of IL-1$\beta$, IL-5, IL-6, IL-7, IL-10, IL-12, IL-13, IL-15, FGFbasic, GM-CSF, IFN-$\gamma$, TNF-$\alpha$, and VEGF as a determination marker.

For a naive patient administered with etanercept (hereinafter, also referred to as an "etanercept therapy naive patient"), it is preferable to use at least one type selected from the group consisting of IL-6, IP-10, IL-2, IL-13, IL-15, sIL-6R, and sTNFRI as a determination marker. It is more preferable to use a combination of IL-2, IL-15, sIL-6R, and sTNFRI as a determination marker.

<Cases where value of disease activity indicator after therapy itself is predicted and determined for biological formulation>

For a tocilizumab therapy naive patient, it is preferable to use at least one type selected from the group consisting of sgp130, IL-8, Eotaxin, IP-10, sTNFRI, sTNFRII, IL-6, and VEGF as a determination marker. It is more preferable to use a combination of sgp130, IL-8, Eotaxin, IP-10, sTNFRI, sTNFRII, and IL-6, or a combination of sgp130, IL-8, Eotaxin, IP-10, sTNFRI, sTNFRII, IL-6, and VEGF as a determination marker.

For a tocilizumab therapy switch patient, it is preferable to use at least one type selected from the group consisting of sgp130, IL-1$\beta$, IL-2, IL-5, IL-15, GM-CSF, IFN-$\gamma$, TNF-$\alpha$, and IP-10 as a determination marker. It is more preferable to use a combination of sgp130, IP-10, and GM-CSF as a determination marker.

For an etanercept therapy naive patient, it is preferable to use at least one type selected from the group consisting of IL-9, IL-6, IL-13, TNF-$\alpha$, and VEGF as a determination marker. It is more preferable to use a combination of IL-9, TNF-$\alpha$, and VEGF, or a combination of IL-6 and IL-13 as a determination marker.

<Cases where possibility of remission (whether remission is reached) by therapy is predicted and determined for biological formulation>

For patients administered with tocilizumab (including both tocilizumab therapy naive patients and tocilizumab therapy switch patients), it is preferable to use at least one type selected from the group consisting of sgp130, IP-10, sTNFRII, IL-6, IL-7, MCP-1 and IL-1$\beta$ as a determination marker. It is more preferable to use at least sgp130, and even more preferable to use a combination of (i) sgp130, (ii) IP-10, (iii) sTNFRII, and (iv) IL-6, IL-7, MCP-1 or IL-1$\beta$. More specifically, for tocilizumab therapy naive patients, a combination of (i) sgp130, (ii) IP-10, (iii) sTNFRII, and (iv) IL-6, IL-7, MCP-1 or IL-1$\beta$ is especially preferable as a determination marker. Further, for tocilizumab therapy switch patients, a combination of (i) sgp130, (ii) IP-10, (iii) sTNFRII, and (iv) IL-6 or IL-1$\beta$ is especially preferable as a determination marker.

For an etanercept therapy naive patient, it is preferable to use at least one type selected from the group consisting of IL-9, TNF-$\alpha$, VEGF, PDGF-bb, and MIP-1$\alpha$ as a determination marker. It is especially preferable to use a combination of IL-9 and TNF-$\alpha$, a combination of VEGF and PDGF-bb, or a combination of MIP-1$\alpha$ and PDGF-bb as a determination marker.

It is known that serum concentration of each of the cytokines, chemokines, and soluble receptors used as a determination marker can be measured by a measurement system utilizing an antigen-antibody reaction such as ELISA. Such measuring kits are commercially available. Thus, the cytokines , chemokines, and soluble receptors can be measured with a known measuring kit by a known method in the determining method of the present invention.

Prediction and determination of therapeutic effect due to biological formulation

A therapeutic effect due to a biological formulation can be predicted and determined based on a measured value of the determination marker. For example, the prediction and determination include a method in which the determination marker is measured in advance for patients in full remission and patients who are not in remission from therapy with a biological formulation; a regression equation of a measured value of the determination marker (explanatory variable) and a therapeutic effect of biological formulation (objective variable) are found by regression analysis; and a measured value of a determination marker of a rheumatoid arthritis patient targeted for determination is applied to said regression equation. When finding a regression equation, it is preferable to use a log value of serum concentration (pg/ml) for the determination markers other than sgp130. For sgp130, a value of serum concentration ($\mu$g/ml) is preferably used. Further, it is preferable that a regression equation is derived by multiple regression analysis. The objective variable in the above-described regression equation may be appropriately determined based on the therapeutic effect to be predicted and determined.

For example, when predicting and determining the level of improvement in a symptom after therapy for a biological formulation, the objective variable may be set to "a value obtained by subtracting a value of a disease activity indicator after a predetermined period of therapy from a value of a disease activity indicator prior to therapy" for analysis by multiple linear regression analysis. For example, when predicting and determining a value of a disease activity indicator after therapy for a biological formulation, the objective variable may be set to "a value of disease activity indicator after a predetermined period of therapy" for analysis by multiple linear regression analysis. In this regard, specific examples of a value of a disease activity indicator include a DAS (Disease activity score)-28 value, CDAI (Clinical Disease Activity Index) value, SDAI (Simple Disease Activity Index) value and the like. A DAS-28 value, CDAI value and SDAI value are correlated with one another and reflect a symptom of rheumatoid arthritis. Thus, any of such disease activity indicator values may be used in the determining method of the present invention. Further, a disease activity indicator used in the determining method of the present invention is not limited to those exemplified above. Indicators that may be newly advocated in the future can be used.

Further, when predicting or determining the possibility of remission due to therapy with a biological formulation (result of whether there is remission or no remission), multiple logistic regression analysis may be used for analysis.

For regression analysis utilizing a measured value of the determination marker as an explanatory variable, a value of a disease activity indicator prior to therapy (DAS-28 value, CDAI value, SDAI value or the like) or a result of evaluation by a Boolean method may be utilized as an explanatory variable.

Hereinafter, therapeutic effects to be predicted and determined are separated into a level of improvement in a symptom after therapy, DAS-28 value after therapy, and possibility of remission to disclose specific methods for the determining method of the present invention. However, the determining method of the present invention should not be interpreted to be limited to the following specific methods.

<Prediction and determination of level of improvement in symptom after therapy>

A level of improvement in a symptom after therapy due to a biological formulation can be predicted and determined by multiple linear regression analysis while setting an objective variable as "a value obtained by subtracting a value of a disease activity indicator after a predetermined period of therapy from a value of a disease activity indicator prior to therapy" and an explanatory variable as "a measured value of the determination marker".

In Examples described below, the following equations (1) and (2) have been discovered as regression equations for predicting and determining a level of improvement in a symptom after 16 weeks of therapy due to a biological formulation (level of improvement in DAS-28 value; DAS-28 value prior to therapy - DAS-28 value after 16 weeks of therapy), separated by the past dosing history of a rheumatoid arthritis patient and type of biological formulation. A level of improvement in a symptom after 16 weeks of therapy can be predicted and determined by finding an objective variable from applying values to one of the following regression equations (1) and (2) depending on the past dosing history of a rheumatoid arthritis patient subjected to determination and type of biological formulation. A level of improvement in a symptom due to a biological formulation is predicted and determined to be large for the patient for larger values of the objective variable calculated by the following regression equation.

[Cases where level of improvement in symptom after 16 weeks of therapy (level of improvement in DAS-28 value; DAS-28 value prior to therapy - DAS28-value after 16 weeks of therapy) is predicted and determined for tocilizumab therapy naive patient]

Determination markers: IL-1$\beta$, IL-7, TNF-$\alpha$, and sIL-6R

Regression equation (1):

Objective function (DAS-28 value prior to therapy - DAS28-value after 16 weeks of therapy) = 5.505 + (-3.618 × A) + (3.255 × B) + (1.475 × C) + (-1.841 × D)

A: log value of serum IL-1β concentration (pg/ml)
B: log value of serum IL-7 concentration (pg/ml)
C: log value of serum TNF-α concentration (pg/ml)
D: log value of serum sIL-6R concentration (pg/ml)

[Cases where level of improvement in symptom after 16 weeks of therapy (level of improvement in DAS-28 value; DAS-28 value prior to therapy - DAS28-value after 16 weeks of therapy) is predicted and determined for etanercept therapy naive patient]

Determination markers: IL-2, IL-15, sIL-6R, and sTNFRI

Regression equation (2):

Objective function (DAS-28 value prior to therapy - DAS-28 value after 16 weeks of therapy) = 7.325 + (-1.567 × E) + (1.632 × F) + (-2.540 × D) + (1.973 × G)

E: log value of serum IL-2 concentration (pg/ml)
F: log value of serum IL-15 concentration (pg/ml)
D: log value of serum sIL-6R concentration (pg/ml)
G: log value of serum sTNFRII concentration (pg/ml)

The regression equations (1) and (2) demonstrate an example of a regression equation used to predict and determine a level of improvement in DAS-28 value after 16 weeks of therapy due to a biological formulation. However, a level of improvement in a CDAI value or an SDAI value after 16 weeks of therapy due to a biological formulation (level of improvement in CDAI value or SDAI value; CDAI value or SDAI value prior to therapy - CDAI value or SDAI value after 16 weeks of therapy) can naturally be predicted and determined by multiple linear regression analysis by the same method using a CDAI value or SDAI value. Further, since a therapeutic effect stabilizes and appears after 16 weeks of therapy by a biological formulation, regression equations for predicting and determining a level of improvement in a symptom after 16 weeks of therapy are shown in the above-described regression equations (1) and (2). Naturally, a level of improvement in a symptom before or after 16 weeks of therapy due to the biological formulations can be predicted and determined by multiple linear regression analysis using the same method.
In Examples described below, the following equations (3)-(7) have been discovered as regression equations for predicting and determining a DAS-28 value of a symptom after 16 weeks of therapy due to a biological formulation, separated by the past dosing history of a rheumatism patient and type of biological formulation. A DAS-28 value after 16 weeks of therapy can be predicted and determined by finding an objective variable from applying values to one of the following regression equations (3)-(7), depending on the past dosing history of a rheumatoid arthritis patient subjected to determination and type of biological formulation. When the objective variable calculated by the following regression equation is 2.3 or less, the patient is predicted and determined to reach remission due to a biological formulation.

[Cases where DAS-28 value after 16 weeks of therapy is predicted and determined for tocilizumab therapy naive patient]

Determination markers: sgp130, IL-8, Eotaxin, IP-10, sTNFRI, sTNFRII, IL-6, and VEGF

Regression equation (3):

Objective function (DAS-28 value after 16 weeks of therapy) = 6.909 + (-5.341 × H) + (3.940 × I)(-1.039 × J) + (-1.002 × K) + (-2.580 × L) + (1.407 × G) + (0.744 × M) + (-0.850 × N)

H: serum sgp130 concentration ($\mu$g/ml)
I: log value of serum IL-8 concentration (pg/ml)
J: log value of serum Eotaxin concentration (pg/ml)
K: log value of serum IP-10 concentration (pg/ml)
L: log value of serum sTNFRII concentration (pg/ml)
G: log value of serum sTNFRII concentration (pg/ml)
M: log value of serum IL-6 concentration (pg/ml)
N: log value of serum VEGF concentration (pg/ml)

[Cases where DAS-28 value after 16 weeks of therapy is predicted and determined for tocilizumab therapy naive patient]

Determination markers: sgp130, IL-8, Eotaxin, IP-10, sTNFRI, sTNFRII, and IL-6

## Regression equation (4):

Objective function (DAS-28 value after 16 weeks of therapy) =
4.731 + (-5.433 × H) + (2.551 × I)(-0.937 × J) + (-1.116 × K)
+ (-2.010 × L) + (1.630 × G) + (0.577 × M)

H: serum sgp130 concentration ($\mu$g/ml)
I: log value of serum IL-8 concentration (pg/ml)
J: log value of serum Eotaxin concentration (pg/ml)
K: log value of serum IP-10 concentration (pg/ml)
L: log value of serum sTNFRII concentration (pg/ml)
G: log value of serum sTNFRII concentration (pg/ml)
M: log value of serum IL-6 concentration (pg/ml)

[Cases where DAS-28 value after 16 weeks of therapy is predicted and determined for tocilizumab therapy switch patient]

Determination markers: sgp130, IP-10, and GM-CSF

## Regression equation (5):

Objective function (DAS-28 value after 16 weeks of therapy) =
2.837 + (-6.037 × H) + (0.714 × K) + (-0.622 × O)

H: serum sgp130 concentration (pg/ml)
K: log value of serum IP-10 concentration (pg/ml)
O: log value of serum GM-CSF concentration (pg/ml)

[Cases where DAS-28 value after 16 weeks of therapy is predicted and determined for etanercept therapy naive patient]

Determination markers: IL-6 and IL-13, DAS-28 value prior to etanercept administration is also used as an explanatory variable

## Regression equation (6):

Objective function (DAS-28 value after 16 weeks of therapy) =
0.081 + (0.522 × a) + (-0.969 × M) + (1.409 × P)

a: DAS-28 value prior to etanercept administration
M: log value of serum IL-6 concentration (pg/ml)
P: log value of serum IL-13 concentration (pg/ml)

[Cases where DAS-28 value after 16 weeks of therapy is predicted and determined for etanercept therapy naive patient]

Determination markers: IL-9, TNF-$\alpha$ and VEGF

```
Regression equation (7):
Objective function (DAS-28 value after 16 weeks of therapy) =
0.703 + (0.646 × S) + (-0.551 × C) + (0.858 × N)
```

S: log value of serum IL-9 concentration (pg/ml)
C: log value of serum TNF-$\alpha$ concentration (pg/ml)
N: log value of serum VEGF concentration (pg/ml)

Since regression equation (7) does not use a DAS-28 value prior to etanercept administration as an explanatory variable, a DAS-28 value after 16 weeks of therapy can be predicted while eliminating a subjective opinion of a physician. Thus, regression equation (7) is considered preferable over regression equation (6).

The regression equations (3)-(7) show examples of a regression equation used to predict and determine a DAS-28 value after 16 weeks of therapy due to a biological formulation. However, a CDAI value or SDAI value itself after 16 weeks of therapy due to a biological formulation can naturally be predicted and determined by multiple linear regression analysis by the same method using a CDAI value or SDAI value. Further, as discussed above, since a therapeutic effect stabilizes and appears after 16 weeks of therapy due to a biological formulation, regression equations for predicting and determining a value of a disease activity indicator after 16 weeks of therapy are shown in the above-described regression equations (3)-(7). However, a value of disease activity indicator prior to or after 16 weeks of therapy due to the biological formulations can naturally be predicted and determined by multiple linear regression analysis using the same method.

In Examples described below, the following equations (8)-(16) have been discovered as regression equations for predicting and determining the possibility of remission (either remission or not in remission) after 16 weeks of therapy due to a biological formulation, separated by the past dosing history of a rheumatoid arthritis patient and type of biological formulation. It is possible to predict and determine whether remission is reached after 16 weeks of therapy by finding the probability (p) of remission after 16 weeks of therapy from applying values to one of the following regression equations (8) - (16) depending on the past dosing history of a rheumatoid arthritis patient subjected to determination and type of biological formulation. The probability of remission estimated from the following regression equations (8) - (16) refers to the probability of a DAS-28 value being 2.3 or less. A p value computed from regression equations (8)-(16) closer to 1 indicates a higher possibility of remission after 16 weeks of therapy. For example, the p value of 0.5 or higher can predict and determine remission and less than 0.5 can predict and determine no remission for convenience's sake. In this regard, a DAS-28 value of 2.3 is used as the boundary between remission and non-remission to enhance the precision of prediction and determination of remission because a CRP value tends to decrease and DAS-28 value may decreases regardless of inflammation by inhibiting IL-6. The value is set at a lower value of DAS-28 value (2.6), which is generally considered the boundary between remission and non-remission.

[Cases where possibility of remission is predicted and determined for tocilizumab therapy naive patient]

Determination markers: sgp130, IP-10, sTNFRII, and IL-6

```
Regression equation (8):
p/(1-p) = exp{(-5.095) + (-36.648 × H) + (-4.004 × K) + (5.632
× G) + (1.658 × M)}
```

p: probability of remission after 16 weeks of therapy
H: serum sgp130 concentration ($\mu$g/ml)
K: log value of serum IP-10 concentration (pg/ml)
G: log value of serum sTNFRII concentration (pg/ml)
M: log value of serum IL-6 concentration (pg/ml)

[Cases where possibility of remission is predicted and determined for tocilizumab therapy naive patient]

Determination markers: sgp130, IP-10, sTNFRII, and IL-7

```
Regression equation (9):
p/(1-p) = exp{(-3.467) + (-42.849 × H) + (-4.430 × K) + (5.736
× G) + (2.705 × B)}
```

p: probability of remission after 16 weeks of therapy
H: serum sgp130 concentration (pg/ml)
K: log value of serum IP-10 concentration (pg/ml)
G: log value of serum sTNFRII concentration (pg/ml)
M: log value of serum IL-7 concentration (pg/ml)

[Cases where possibility of remission is predicted and determined for tocilizumab therapy naive patient]

Determination markers: sgp130, IP-10, sTNFRII, and MCP-1

```
Regression equation (10):
p/(1-p) = exp{(-2.834) + (-38.721 × H) + (-4.664 × K) + (5.369
× G) + (2.502 × B)}
```

p: probability of remission after 16 weeks of therapy
H: serum sgp130 concentration (pg/ml)
K: log value of serum IP-10 concentration (pg/ml)
G: log value of serum sTNFRII concentration (pg/ml)
P: log value of serum MCP-1 concentration (pg/ml)

[Cases where possibility of remission is predicted and determined for tocilizumab therapy naive patient]

Determination markers: sgp130, IP-10, sTNFRII, and IL-1β

```
Regression equation (11):
p/(1-p) = exp{(-1.269) + (-39.538 × H) + (-3.807 × K) + (5.086
× G) + (1.647 × A)}
```

p: probability of remission after 16 weeks of therapy
H: serum sgp130 concentration (μg/ml)
K: log value of serum IP-10 concentration (pg/ml)
G: log value of serum sTNFRII concentration (pg/ml)
A: log value of serum IL-1β concentration (pg/ml)

[Cases where possibility of remission is predicted and determined for tocilizumab therapy switch patient]

Determination markers: sgp130, IP-10, sTNFRII, and IL-6

```
Regression equation (12):
p/(1-p) = exp{(-10.935) + (-29.051 × H) + (4.466 × K) + (2.067
× G) + (-2.757 × M)}
```

p: probability of remission after 16 weeks of therapy
H: serum sgp130 concentration (μg/ml)
K: log value of serum IP-10 concentration (pg/ml)
G: log value of serum sTNFRII concentration (pg/ml)
M: log value of serum IL-6 concentration (pg/ml)

[Cases where possibility of remission is predicted and determined for tocilizumab therapy switch patient]

Determination markers: sgp130, IP-10, sTNFRII, and IL-1β

Regression equation (13):
$$p/(1-p) = \exp\{(-9.671) + (-27.150 \times H) + (3.205 \times K) + (1.914 \times G) + (-2.540 \times A)\}$$

p: probability of remission after 16 weeks of therapy
H: serum sgp130 concentration (pg/ml)
K: log value of serum IP-10 concentration (pg/ml)
G: log value of serum sTNFRII concentration (pg/ml)
A: log value of serum IL-1β concentration (pg/ml)

[Cases where possibility of remission is predicted and determined for etanercept therapy naive patient]

Determination markers: VEGF and PDGF-bb, DAS-28 value prior to etanercept administration is also used as an explanatory variable.

Regression equation (14):
$$p/(1-p) = \exp\{(-19.058) + (1.390 \times a) + (-2.763 \times E) + (4.962 \times Q)$$

p: probability of remission after 16 weeks of therapy
a: DAS-28 value prior to etanercept administration
E: log value of serum VEGF concentration (pg/ml)
Q: log value of serum PDGF-bb concentration (pg/ml)

[Cases where possibility of remission is predicted and determined for etanercept therapy naive patient]

Determination markers: MIP-1α and PDGF-bb, DAS-28 value prior to etanercept administration is also used as an explanatory variable.

Regression equation (15):
$$p/(1-p) = \exp\{(-18.491) + (1.107 \times a) + (-1.808 \times R) + (3.930 \times Q)\}$$

p: probability of remission after 16 weeks of therapy
a: DAS-28 value prior to etanercept administration
R: log value of serum MIP-1α concentration (pg/ml)
Q: log value of serum PDGF-bb concentration (pg/ml)

[Cases where possibility of remission is predicted and determined for etanercept therapy naive patient]

Determination markers: IL-9 and TNF-α

### Regression equation (16):

$$p/(1-p) = \exp\{(-1.004) + (1.711 \times S) + (-1.031 \times C)\}$$

p: probability of remission after 16 weeks of therapy
S: log value of serum IL-9 concentration (pg/ml)
C: log value of serum TNF-$\alpha$ concentration (pg/ml)

Since the regression equation (16) does not use a DAS-28 value prior to etanercept administration as an explanatory variable, the possibility of remission can be predicted while eliminating a subjective opinion of a physician. Thus, regression equation (16) is considered preferable over regression equations (14) and (15).

The regression equations (6)-(16) show examples of a regression equation for predicting and determining the possibility of remission after 16 weeks of therapy, with a DAS-28 value after 16 weeks of therapy of 2.3 or lower considered remission and the value over 2.3 as non-remission. However, the possibility of remission after 16 weeks of therapy due to a biological formulation can naturally be predicted and determined by multiple logistic regression analysis with the same method using a CDAI value or SDAI value. Further, as discussed above, since a therapeutic effect stabilizes and appears after 16 weeks of therapy by a biological formulation, regression equations for predicting and determining the possibility of remission after 16 weeks of therapy are shown in the above-described regression equations (6) - (16) . However, the possibility of remission prior to or after 16 weeks of therapy due to a biological formulation can be predicted and determined by multiple logistic regression analysis using the same method.

Selection of biological formulation to be administered

The determining method of the present invention can predict the therapeutic effectiveness of a biological formulation prior to the administration thereof. Thus, the method can be utilized in selecting the optimal biological formulation that should be administered prior to starting therapy.

For example, for a level of improvement in a symptom after therapy of a naive patient, cases in which tocilizumab is administered and cases in which etanercept is administered are each predicted by the aforementioned method and a biological formulation with a higher level of improvement is selected, so that an optimal biological formulation can be administered to the patient. Specifically, a level of improvement in a symptom after therapy with tocilizumab therapy, which is predicted by using regression equation (1) is compared to a level of improvement in a symptom after therapy with etanercept therapy, which is predicted by using regression equation (2), so that the biological formulation with a higher level of improvement can be selected as the optimal biological formulation.

For example, for a DAS-28 value after 16 weeks of therapy of a naive patient, cases in which tocilizumab is administered and cases in which etanercept is administered are each predicted by the aforementioned method and a biological formulation with a lower DAS-28 value after 16 weeks of therapy is selected so that an optimal biological formulation can be administered to the patient. Specifically, a DAS-28 value after 16 weeks of therapy with tocilizumab therapy, which is predicted by using one of regression equations (3)-(5) is compared to a DAS-28 value after 16 weeks of therapy with etanercept therapy, which is predicted by using regression equation (6) or (7), so that the biological formulation with a smaller DAS-28 value can be selected as the optimal biological formulation.

For example, for the possibility of remission of a naive patient, cases in which tocilizumab is administered and cases in which etanercept is administered are each predicted by the aforementioned method to select a biological formulation with a higher possibility of remission, so that the optimal biological formulation can be administered to the patient. Specifically, the possibility of remission with tocilizumab therapy, which is predicted by using one of regression equations (8)-(11), is compared to the possibility of remission , which is predicted by using one of regression equations (14)-(16), so that the biological formulation with a higher possibility of remission can be selected as the optimal biological formulation.

2. Diagnostic agent

The present invention further provides a diagnostic agent for carrying out the above-described detection method. Specifically, the diagnostic agent of the present invention is a diagnostic agent for determining the effectiveness of therapy due to a biological formulation targeting an inflammatory cytokine for a rheumatoid arthritis patient, characterized by comprising a reagent capable of detecting at least one type of determination marker selected from the group consisting of sgp130, IP-10, sTNFRI, sTNFRII, GM-CSF, IL-1$\beta$, IL-2, IL-5, IL-6, IL-7, IL-8, IL-9, IL-10, IL-12, IL-13, IL-15, Eotaxin, VEGF, MCP-1, TNF-$\alpha$, IFN-$\gamma$, FGFbasic, PDGF-bb, sIL-6R, and MIP-1$\alpha$.

The determination marker can be measured by a measurement system utilizing an antigen-antibody reaction such as ELISA. Specific examples of reagents capable of detecting the determination marker include antibodies that can specifically bind to the determination marker and fragments thereof. Further, antibodies that can specifically bind to the determination marker may be bound on a suitable support to be provided as an antibody array.

Furthermore, the diagnostic agent used in the present invention may comprise a reagent (secondary antibody, color producing substance or the like) required for detecting the determination marker by an antigen-antibody reaction.

Examples

Hereinafter, the present invention is disclosed in detail while using Examples. However, the present invention is not limited thereby.

1. Patient and Experimental Method

(Patient)

Hereinafter, a rheumatism patient who has not received anti-cytokine therapy (administration of infliximab, etanercept, adalimumab, tocilizumab or the like) in the past is referred to as a naive patient, and a rheumatism patient who has received anti-cytokine therapy in the past is referred to as a switch patient.

155 rheumatoid arthritis patients, to whom methotrexate therapy was ineffective, were registered at the Higashihiroshima Memorial Hospital from March 2008 to June 2013. Among the 155 patients, 98 patients received therapy with tocilizumab and the remaining 57 patients received therapy with etanercept. Among the 98 patients who received therapy with tocilizumab, 58 patients were naive patients who had not previously received anti-cytokine therapy and 40 patients were switch patients who had previously received anti-cytokine therapy 1-3 times. Among 57 patients who received etanercept therapy, 49 patients were naive patients who had not previously received anti-cytokine therapy, and the remaining 8 patients were switch patients who had previously received anti-cytokine therapy. Informed consent was obtained prior to receiving a blood sample supply from all patients. Further, the tests were conducted with permission prior to the study from the ethics committee of the Higashihiroshima Memorial Hospital.

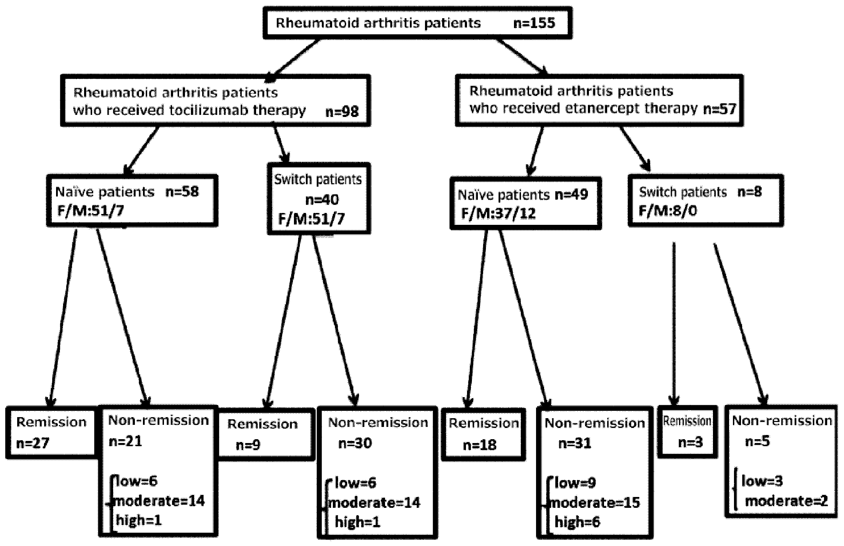

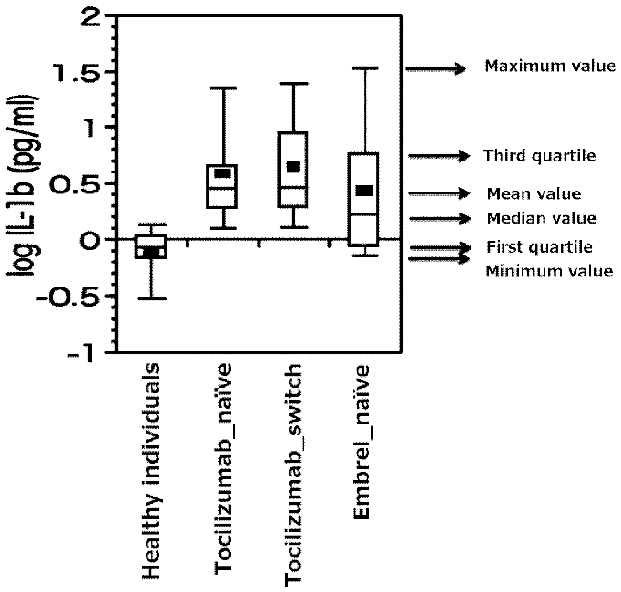

Table 1 shows the clinical baseline individual group statistics for group of individuals and clinical diagnosis. Further, Figure 1 shows a trial profile of patients receiving therapy with tocilizumab and patients receiving therapy with etanercept. Figures **2-1** to **2-4** show serum concentration of cytokine/chemokine/soluble receptor prior to therapy. 9 naive patients (8 patients who suffered from side effects or other diseases and 1 patient for whom data could not be obtained for the entire 16 weeks) among patients treated with tocilizumab were eliminated. Further, 1 switch patient suffering from a side effect (patient for whom data could not be obtained for the entire 16 weeks) among patients treated with tocilizumab was eliminated. There was hardly any difference in DAS-28 value, CRP, swollen joint count, tender joint count, Stage, and Class among the groups (Table 1). Further, the duration of disease was shorter for patients treated with etanercept in comparison to patients treated with tocilizumab (Table 1).

In order to create a baseline concentration of cytokines, serum was collected from healthy individuals (56 individual; 20 males and 36 females) without a history of suffering from hepatitis C or cancer. The healthy individuals underwent medical examination by the Louis Pasteur Center for Medical Research or the Higashihiroshima Memorial Hospital and informed consent was received in writing from the healthy individuals. The baseline concentration was used to find a distribution pattern of cytokines/chemokines/soluble receptors.

(Experimental Method)

Prior to therapy, concentrations of cytokines, chemokines, and soluble receptors in the serum of rheumatoid arthritis patients were measured.

Figure 1 shows clinical results for naive patients and switch patients administered with 8 mg/kg tocilizumab or 50 mg/kg etanercept once every 4 weeks. After 16 weeks of therapy (after 4 administrations), a therapeutic effect was determined based on DAS-28-CRP values and whether the patient is in remission or non-remission. Results for non-remission were further classified into low, medium and high based on DAS-28-CRP values of the patients. DAS-28-ESR values are extensively used to determine the symptom of rheumatoid arthritis patients. However, it is reported that DAS-28-CRP values are almost interchangeable with DAS-28-ESR values and the same results are derived therefrom (Ann Rheum Dis. 2007, March 407-409 Comparison of Disease Activity Score (DAS)28-erythrocyte Sedimentation rate and DAS-C-reactive protein threshold votes. Inoue E, Yamanaka H, et al.)

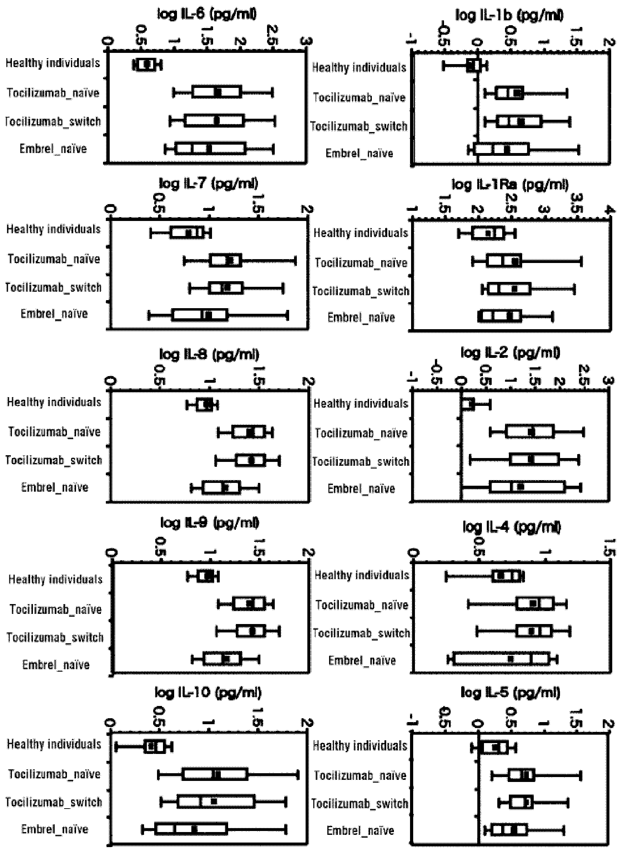

In the present tests, DAS-28-CRP values were used to determine the symptoms of rheumatoid arthritis patients. Remission was classified as DAS-28-CRP value < 2.3 and non-remission was classified as DAS-28-CRP value $\geq$ 2.3. Furthermore, DAS-28-CRP classification system developed by Inoue et al was used to classify non-remission patients as low (DAS-28-CRP value = 2.3-2.6), medium (DAS-28-CRP value = 2.7-4.1) and high (DAS-28-CRP value > 4.1) depending of the severity of the symptoms. To obtain consistent determination of symptoms, the same physician at the Higashihiroshima Memorial Hospital determined the final symptoms of all patients. Further, Figure 3 shows detailed clinical results of each patient shown in Figure 1, i.e., results of determining DAS-28-CRP values prior to therapy and after 16 weeks of therapy for naive patients who received tocilizumab therapy, switch patients who received tocilizumab therapy, and naive patients who received etanercept therapy. Hereinafter, DAS-28-CRP values may be denoted simply as DAS-28 values.

(Analysis of cytokine/chemokine/soluble receptor)

For all measurements of cytokines, a multiplex cytokine array system (Bio-Plex 200, Bio-Rad Laboratories) was used in accordance with the product protocol thereof. Serum for all patients and healthy individuals were collected by 10 minutes of centrifugation (1600xg). All serum samples were stored at -80° C. Bio-Plex Human Cytokine 27-Plex Panel is configured such that 27 types of cytokines (IL-1β, IL-1RA, IL-2, IL-4, IL-5, IL-6, IL-7, IL-8, IL-9, IL-10, IL-12 (p70), IL-13, IL-15, IL-17, basic FGF, eotaxin, G-CSF, GM-CSF, IFN-γ, IP-10, MCP-1, MIP-1α, MIP-1β, PDGF-bb, RANTES, TNF-α, and VEGF) can be analyzed. In addition thereto, sIL-6R, sgp130, sTNF-RI and sTNF-RII were also analyzed (Milliplex®MAP, Human Soluble Cytokine Receptor Panel: Millipore Co. MA). In the present test, concentrations of cytokines, chemokines, and soluble receptors of 56 healthy individuals were simultaneously measured to find the distribution patterns thereof. Bio-Plex Manager software version 5.0 was used to conduct data collection and analysis.

(Statistical Analysis)

The distribution of cytokine/chemokine values in healthy individuals was analyzed. The log values of the values of the concentration (pg/ml) of cytokines, chemokines, and soluble receptors, other than sgp130, were used for the analysis. The values of concentration (pg/ml) were directly used for sgp130.

First, simple linear regression analysis and multiple linear regression analysis were performed to investigate the association between cytokine/chemokine/soluble receptor concentration or clinical test values and values obtained by subtracting DAS-28 values after 16 weeks from DAS-28 values of patients at 0 weeks. Next, DAS-28 values after 16 weeks were estimated from a value computed from regression introduced with the clinical test values. Furthermore, simple logistic regression analysis and multiple logistic regression analysis were preformed to analyze the relationship between serum cytokine concentration and remission or non-remission. The resulting parameter p value of $<0.05$ indicates the presence of a significant difference. All statistical analysis was conducted by using the JMP 9.0 software.

2. Results

(Clinical evaluation)

Tables 1 and 2 show clinical baseline individual group statistics, clinical diagnosis and cytokine/chemokine/soluble receptor characteristics. Figures 2-1 to 2-4 shows clinical baseline individual group statistics for healthy individuals and rheumatoid arthritis patients with respect to serum concentrations of cytokines/chemokines/soluble receptors prior to therapy. In Table 1, Stage and Class are results of determination with respect to functional classification criteria for rheumatoid arthritis based on Steinbrocker (1949) classification (I-IV; Steinbrocker O et al: Therapeutic criteria in rheumatoid arthritis. JAMA 140:659,1949) and Hochberg (1992) classification (I-IV; Hochberg MC et al, The American College of Rheumatology 1991 revised criteria for the Classification of global functional status in rheumatoid arthritis. Arthritis and Rheumatism, 35:498-502, 1992), respectively. The results indicate that there is no clinically significant difference among the three rheumatism patient groups and the serum concentrations of cytokines/chemokines/soluble receptors in most rheumatism patients are significantly higher in comparison to healthy individuals.

[Table 1]

Basic information data for patients

| | Naïve patients who received tocilizumab therapy | | | | | | Switch patients who received tocilizumab therapy | | | | | | Naïve patients who received etanercept therapy | | | | | |
|---|---|---|---|---|---|---|---|---|---|---|---|---|---|---|---|---|---|---|
| Clinical parameter | n=48 | | | 75% quartile | Median | 25% quartile | n=40 | | | 75% quartile | Median | 25% quartile | n=43 | | | 75% quartile | Median | 25% quartile |
| Age (years) | 59,4 | ± | 1,8 | 68,5 | 62 | 55,25 | 56,75 | ± | 1,84 | 65,25 | 59 | 49,5 | 59,2 | ± | 1,91 | 68 | 61 | 52 |
| Duration of disease (years) | 10,6 | ± | 1,2 | 15,5 | 10 | 3,6 | 10,85 | ± | 1,17 | 14,75 | 10 | 5,47 | 7,9 | ± | 8,4 | 11 | 5 | 2,5 |
| WBC ( /µl) | 8254 | ± | 424 | 10247 | 7930 | 5812 | 8192 | ± | 371 | 9857,5 | 7670 | 6770 | 8173 | ± | 456 | 9890 | 7470 | 6200 |
| Fe | 41,5 | ± | 4,45 | 53 | 39 | 23,5 | 57,48 | ± | 7,29 | 86 | 54 | 21 | 54,2 | ± | 5,38 | 67 | 46 | 27 |
| Ferritin | 94,8 | ± | 12,8 | 114,9 | 81,3 | 40,5 | 64,28 | ± | 9,05 | 100,55 | 46,2 | 28,6 | 140 | ± | 28,4 | 206,25 | 57,7 | 35,1 |
| RBC | 384 | ± | 8,4 | 405,2 | 388,5 | 362,2 | 409,8 | ± | 8,67 | 448,25 | 402 | 374,5 | 403 | ± | 8,07 | 444 | 400 | 362 |
| Hb | 11 | ± | 0,23 | 11,8 | 11,3 | 10,4 | 11,77 | ± | 0,29 | 13,12 | 11,6 | 10,55 | 11,9 | ± | 0,24 | 13,3 | 12,2 | 10,8 |
| Ht | 35,4 | ± | 0,6 | 37,4 | 35,7 | 33,8 | 37,56 | ± | 0,82 | 35,4 | 28,1 | 23,3 | 37,3 | ± | 0,65 | 40,9 | 38 | 34,4 |
| Plt | 32,3 | ± | 1,3 | 38,5 | 32,8 | 25,7 | 29,03 | ± | 1,38 | 35,4 | 28,1 | 23,32 | 29,7 | ± | 1,51 | 39,2 | 26,5 | 21,8 |
| CRP(QT/QL)(mg/dl) | 3,5 | ± | 0,67 | 4,5 | 2,29 | 0,8 | 2,37 | ± | 0,49 | 2,94 | 1,21 | 0,44 | 2,75 | ± | 0,41 | 4,54 | 1,62 | 0,6 |
| DAS28-CRP | 4,64 | ± | 0,19 | 5,63 | 4,39 | 3,49 | 4,39 | ± | 0,14 | 5,11 | 4,42 | 3,85 | 4,68 | ± | 0,18 | 5,4 | 4,73 | 4 |
| RF (U/ml) | 155 | ± | 31 | 231 | 70,5 | 16,25 | 89,17 | ± | 14,5 | 148 | 52 | 17 | 188 | ± | 53,2 | 185 | 80 | 27 |
| VAS | 53,3 | ± | 3,66 | 71,25 | 50 | 30 | 57,3 | ± | 3,63 | 75 | 50 | 40 | 57,1 | ± | 4,33 | 76 | 50 | 44,25 |
| Swollen joint count | 7,04 | ± | 0,89 | 9 | 5 | 2 | 5,07 | ± | 0,46 | 7,75 | 5 | 2 | 6,39 | ± | 0,7 | 9 | 6 | 3 |
| Tender joint count | 6,25 | ± | 0,92 | 9 | 4 | 2 | 5,25 | ± | 0,42 | 6,75 | 5 | 3,75 | 6,58 | ± | 0,78 | 8 | 5 | 3 |
| Stage | 2,93 | ± | 0,15 | 4 | 3 | 2 | 3,7 | ± | 0,08 | 4 | 4 | 3 | 2,83 | ± | 0,16 | 4 | 3 | 2 |
| Class | 2,04 | ± | 0,06 | 2 | 2 | 1,8 | 2,3 | ± | 0,07 | 3 | 2 | 2 | 2,16 | ± | 0,09 | 3 | 2 | 1 |

WBC — White blood cell count ($\times 10^3/\mu\ell$)

Fe — Serum iron ($\mu g/d\ell$)

Ferritin — Ferritin ($ng/d\ell$) CLIA method

RBC — Red blood cell count ($\times 10^6/\mu\ell$)

Hb — Hemoglobin value ($g/d\ell$)

Ht — Hematocrit value (%)

Plt — Platelet count ($\times 10^3/\mu\ell$)

CRP — C-reactive protein ($mg/d\ell$)

DAS28-CRP — Disease activity score obtained by changing a variable for the erythrocyte sedimentation rate to a variable for CRP → DAS is an evaluation method recommended by EULAR (The European League Against Rheumatism). Absolute values of disease activity are calculated.

RF — Rheumatoid factor concentration ($IU/m\ell$) — DAS28 assessment is narrowed down to 28 joints.

VAS — Level of pain with 100 mm as the maximum pain experienced up to date (mm) — DAS28 is calculated with the formula* by measuring the following 4 items

Stage — Functional classification criteria for rheumatoid arthritis (mainly level of radiological progression) (I-IV) — (1) Tender joints / (2) Swollen joints

Class — Functional classification criteria for rheumatoid arthritis (mainly level of difficulty in terms of daily living) (I-IV) — (3) Patient global health condition (in terms of VAS) / (4) CRP or ESR

Formula*    $DAS28 = 0.56 \times \sqrt{T28} + \sqrt{s28} + 0.7 \times in(CRP) + 0.014 \times GH$

<References>   Van Der Heijde DMFM et al. Ann Rheum Dis 49, 916-920, 1990

Van Der Heijde DMFM et al. Ann Rheum Dis 51, 177-181, 1992

49 naive patients received 16 weeks of tocilizumab therapy. 56% of the patients thereof (27 patients) exhibited remission and the rest of the 21 patients exhibited non-remission (Figure 1). Furthermore, 39 switch patients received 16 weeks of tocilizumab therapy, of which 9 patients exhibited remission and the rest of the 30 patients exhibited non-remission.

Further, 49 naive patients received 16 weeks of etanercept therapy. 18 patients thereamong exhibited remission and the remaining 31 patients exhibited non-remission (Figure 1). In the present test, the number of switch patients was extremely low. Thus, only naive patients were used in the analysis for etanercept therapy.

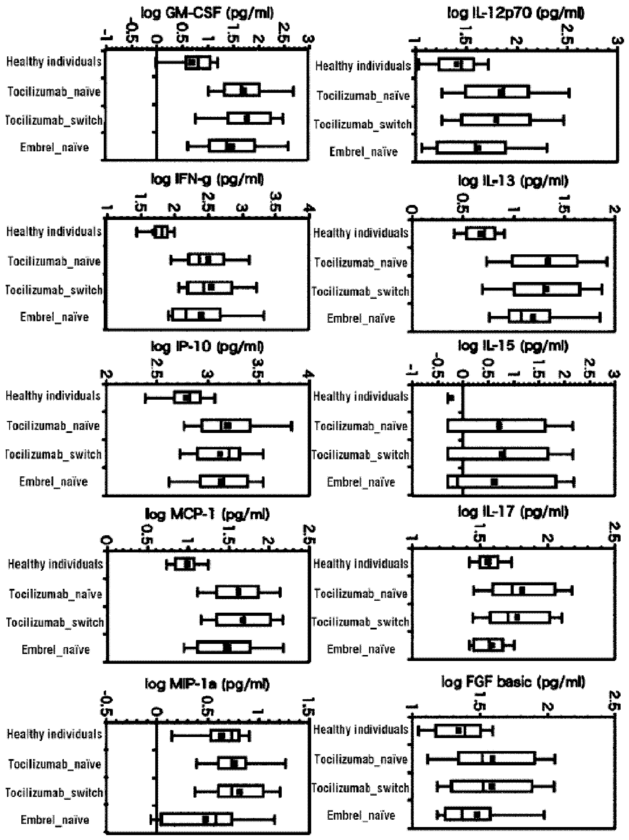

Figure **4** shows the relationship between a DAS-28 value prior to therapy and a value obtained from subtracting a DAS-28 value after 16 weeks from the DAS-28 value prior to therapy (PreDAS-28score - 16W DAS-28 score) in a naive patient who has received tocilizumab therapy (PreDAS-28 score). According to Figure **4,** improvement in DAS-28 values was observed after tocilizumab therapy in most naive patients.

(Search for biomarkers based on DAS-28 value after 16 weeks of therapy by using serum concentration of cytokines/chemokines/soluble receptors in rheumatism patient prior to therapy)

According to the current results, about 55% of naive patients and about 23% of switch patients are expected to exhibit remission after tocilizumab therapy. Although the final symptoms are not identical, some improvement in the symptom is observed in about 45% of naive patients and about 77% of switch patients. Further, about 36.7% of naive patients are expected to exhibit remission after etanercept therapy. In addition, some improvement in the symptom is observed after etanercept therapy in about 60% of the remaining patients.

It has been reported by Pers Y.M. et al (Rheumatology (2013) doi: 10.1093/rheumatology/ket301 First published online: September 19, 2013) that when DAS-28 values after 12-24 weeks of tocilizumab therapy were assessed from general medical examination, 40% of patients exhibited remission, and the main prediction markers were young patients, high CRP value, and patients without a cardiovascular disorder. Further, Koike T (J. Rheumatology, November 2013) et al reported 47.6% remission with respect to DAS28-ESR after 28 weeks of tocilizumab therapy. Meanwhile for etanercept, it is reported by Markenson JA et al (J. Rheumatology, July 2011, p. 1273-81) in the RADIUS study and Curitis JR et al (Ann RheumDis. 2012. 71. 206-212) in the TEMPO study that patients achieving a low disease activity of DAS-28 value $\leq$ 3.2 after 52 weeks and remission were 53%, and 63% when methotrexate is added. Further, Koike T et al (J. Rheumatology, October 2013, p. 1658-1668) have demonstrated that therapy of etanercept adding with methotrexate, when assessing DAS-28 values after 24 weeks, was more effective than therapy with etanercept alone or therapy adding an anti-rheumatism agent (DMARD) other than methotrexate. Furthermore, it is reported by Cannon GW et al (Clin Exp Rheumatol. November 2013) that 35% reached remission in a 3 year observation from TEMPO and RADIUS studies. Furthermore, Cannon GW et al demonstrated that patients with low disease activity are more likely exhibit remission. However, these reports do not reveal a marker for predicting and determining a therapeutic effect due to a biological formulation.

In this regard, serum concentrations of cytokines/chemokines were used to investigate whether it is possible to estimate the level of improvement in rheumatoid arthritis based on DAS-28 values after 16 weeks of therapy.

Figures **2-1** to **2-4** show comparisons of baseline individual group statistics of cytokines/chemokines/soluble receptors prior to therapy for healthy individuals and three groups (naive patients who received tocilizumab therapy, switch patients who received tocilizumab therapy, naive patients who received etanercept therapy). As can be seen from Figures **2-1** to **2-4**, serum concentration other than those for sgp130, sIL-6R and sTNFRI in rheumatoid arthritis patients were significantly higher in comparison to healthy individuals. Further, serum concentrations of cytokines/chemokines were lower in naive patients who received etanercept therapy in comparison to naive patients who received tocilizumab therapy. Further, it was revealed from this result that naive patients who received tocilizumab therapy have a higher CRP value prior to therapy in comparison to naive patients who received etanercept therapy.

Simple linear regression analysis was performed to find the cytokine/chemokine involved in the level of improvement in DAS-28 values. The level of improvement in a DAS-28 value (DAS-28 value prior to therapy - DAS-28 value after 16 weeks of therapy) was used as an objective variable and serum concentration of cytokines/chemokines/soluble receptors were used directly, or by converting into a log value, as an independent variable. The results are shown in Table 2. As shown in Table 2, logIL-7, logIL-8, logIL-12, logIL-13, logIP-10 and logVEGF exhibiting $p < 0.05$ significantly matched the level of improvement in DAS-28 values in naive patients who received tocilizumab therapy. Further, for switch patients who received tocilizumab therapy, logIL-1$\beta$, logIL-5, logIL-6, logIL-7, logIL-10, logIL-12, logIL-13, logIL-15, logFGF, logGM-CSF, logIFN-$\gamma$, logTNF-$\alpha$ and logVEGF significantly matched the level of improvement in DAS-28 values. Meanwhile, logIL-6 and logIP-10 significantly matched the level of improvement in DAS-28 values for naive patients who receivedetanercept therapy.

## Simple linear regression analysis Level of improvement in DAS-28

### Objective variable: DAS-28 Improvement (=0 week DAS-28 value - 16 week DAS-28 value)

| Cytokine/Chemokine | Naïve patients who received tocilizumab therapy | | Switch patients who received tocilizumab therapy | | Naïve patients who received etanercept therapy | |
|---|---|---|---|---|---|---|
| | Estimates | p value | Estimates | p value | Estimates | p value |
| logHu IL-1b | 0,211 | 0,513 | 0,944 | 0,006 | 0,290 | 0,321 |
| logHu IL-1ra | 0,298 | 0,189 | 0,386 | 0,142 | 0,240 | 0,341 |
| logHu IL-2 | 0,294 | 0,213 | 0,522 | 0,278 | 0,240 | 0,246 |
| logHu IL-4 | 0,836 | 0,158 | 0,789 | 0,238 | 0,453 | 0,344 |
| logHu IL-5 | 0,534 | 0,133 | 1,337 | 0,003 | 0,198 | 0,598 |
| logHu IL-6 | 0,519 | 0,067 | 0,701 | 0,016 | 0,572 | 0,040 |
| logHu IL-7 | 0,890 | 0,035 | 1,204 | 0,011 | 0,207 | 0,576 |
| logHu IL-8 | 1,603 | 0,043 | 0,447 | 0,439 | 0,743 | 0,230 |
| logHu IL-9 | 0,345 | 0,136 | 0,327 | 0,169 | 0,182 | 0,460 |
| logHu IL-10 | 0,589 | 0,058 | 0,860 | 0,011 | 0,054 | 0,865 |
| logHu IL-12 | 0,918 | 0,010 | 1,059 | 0,008 | 0,004 | 0,990 |
| logHu IL-13 | 0,755 | 0,036 | 0,930 | 0,016 | -0,023 | 0,959 |
| logHu IL-15 | 0,276 | 0,099 | 0,433 | 0,010 | 0,306 | 0,073 |
| logHu IL-17 | 0,438 | 0,453 | 0,536 | 0,431 | -0,174 | 0,873 |
| logHu Eotaxin | 0,574 | 0,084 | 0,753 | 0,068 | 0,570 | 0,122 |
| logHu FGF basic | 0,333 | 0,396 | 0,978 | 0,045 | 0,276 | 0,589 |
| logHu G-CSF | 0,290 | 0,576 | 1,331 | 0,084 | 0,347 | 0,479 |
| logHu GM-CSF | 0,143 | 0,573 | 0,692 | 0,002 | 0,115 | 0,675 |
| logHu IFN-g | 0,297 | 0,397 | 1,069 | 0,005 | 0,289 | 0,381 |
| logHu IP-10 | 1,119 | 0,009 | 0,582 | 0,258 | 0,969 | 0,049 |
| logHu MCP-1 | 0,610 | 0,135 | 0,543 | 0,208 | 0,659 | 0,103 |
| logHu MIP-1a | 0,962 | 0,057 | 0,751 | 0,099 | 0,451 | 0,262 |
| logHu PDGF-bb | 0,859 | 0,104 | 0,256 | 0,650 | -0,845 | 0,323 |
| logHu MIP-1b | 1,108 | 0,108 | 0,124 | 0,842 | 0,461 | 0,258 |
| logHu RANTES | 0,664 | 0,144 | 0,297 | 0,519 | -0,826 | 0,348 |
| logHu TNF-a | 0,364 | 0,187 | 0,810 | 0,010 | 0,398 | 0,099 |
| logHu VEGF | 0,996 | 0,007 | 0,899 | 0,028 | 0,208 | 0,626 |
| sgp130 | 0,000 | 0,216 | 0,000 | 0,382 | 0,000 | 0,669 |
| logHu-sIL-6R | -0,981 | 0,292 | 0,783 | 0,370 | -0,798 | 0,332 |
| logHu-sTNFRI | -0,360 | 0,617 | -0,555 | 0,439 | 0,131 | 0,828 |
| logHu-sTNFRII | -0,855 | 0,312 | -0,111 | 0,689 | -0,083 | 0,863 |
| CRP | 0,081 | 0,025 | 0,064 | 0,265 | 0,014 | 0,841 |
| 0wDAS28-CRP | 0,693 | <0.0001 | 0,741 | <0.0001 | 0,597 | <0.0001 |
| MMP | 0,001 | 0,384 | 0,002 | 0,058 | -0,001 | 0,473 |
| RF | 0,001 | 0,090 | 0,004 | 0,039 | 0,001 | 0,220 |
| VAS | 0,029 | <0.0001 | 0,020 | 0,024 | 0,025 | <0.0001 |
| Swollen joint count | 0,082 | 0,002 | 0,133 | 0,026 | 0,102 | 0,009 |
| Tender joint count | 0,106 | <0.0001 | 0,166 | 0,009 | 0,121 | 0,000 |

[Table 2]

Multiple linear regression analysis was performed to find the correlation between the level of improvement in DAS-28 value and cytokine/chemokine/soluble receptor concentration. As a result, it was found by phased multiple regression analysis that a combination of logIL-1β, logIL-7, logTNF-α and logsIL-6R is significantly correlated with the level of improvement in DAS-28 values in naive patients who received tocilizumab therapy (Table 3).

Meanwhile, a combination of logIL-2, logIL-15, logIL-6R, and logTNFRI was found to have significant correlation with the level of improvement in DAS-28 values in naive patients who received etanercept therapy (Table 4).

[Table 3]

Multiple linear regression analysis on naïve patients who received tocilizumab therapy
Level of improvement in DAS-28

Objective variable : DAS-28 improvement
(=0 week DAS-28 value - 16 week DAS-28 value)

| Naïve patients who received tocilizumab therapy | | |
|---|---|---|
| Multiple regression analysis  (Objective value=0w~16wDAS28) | | |
| R^2 | 0.376 | |
| ANOVA(Analysis of variance) | p=0.0004 | |
| Cytokine/Chemokine/soluble receptor | Estimate | p value |
| intercept | 5.505 | 0.1216 |
| logHu IL-1b | -3.618 | 0.0002 |
| logHu IL-7 | 3.255 | 0.0002 |
| logHu TNF-a | 1.475 | 0.0221 |
| logHu-sIL-6R | -1.814 | 0.0264 |

[Table 4]

Naïve patients who received etanercept therapy
Multiple linear regression analysis   Level of improvement in DAS-28

Objective variable : DAS-28 improvement
(=0 week DAS-28 value - 16 week DAS-28 value)

| Naïve patients who received etanercept therapy | | |
|---|---|---|
| Multiple regression analysis  (Objective value=0w~16wDAS28) | | |
| R^2 | 0.343 | |
| ANOVA(Analysis of variance) | p=0.0037 | |
| Cytokine/Chemokine/soluble receptor | Estimate | p value |
| intercept | 7.325 | 0.0231 |
| logHu IL-2 | -1.567 | 0.0058 |
| logHu IL-15 | 1.632 | 0.0008 |
| logHusIL-6R | -2.540 | 0.0130 |
| logHu-sTNFRI | 1.973 | 0.0115 |

Simple linear regression analysis was performed to find the cytokine/chemokine/soluble receptor involved in the final assessment of a DAS-28 value after 16 weeks of therapy (16wDAS28). The DAS-28 value after 16 weeks of therapy was used as an objective variable and serum concentration of cytokines/chemokines/soluble receptors were used directly, or by converting into a log value, as an independent variable. As shown in Table 5, sgp130 exhibiting $p < 0.05$ significantly matched DAS-28 values after 16 weeks of therapy in naive patients who received tocilizumab therapy. Further, for switch patients who received tocilizumab therapy, logIL-1β, logIL-2, logIL-5, logIL-15, logGM-CSF, logIFN-γ, logTNF-α and sgp130 significantly matched DAS-28 values after 16 weeks of therapy. Meanwhile, logIL-9 significantly matched DAS-28 values after 16 weeks of therapy for naive patients who received etanercept therapy.

[Table 5]

Simple linear regression analysis 16-week DAS-28

Objective variable: 16-week DAS-28

Simple linear regression anlysis of cytokine /chemokine /soluble receptor based on DAS-28 16w

Simple linear regression anlysis were performed to find the parameters related to 16wDAS-28 (=16wDAS28).

| | | | Naïve Tocilizumab Therapy | | Switch Tocilizumab Therapy | | Naïve Etanercept Therapy | |
|---|---|---|---|---|---|---|---|---|
| | | | Tocilizumab naïve | | Tocilizumab switch | | Etanercept naïve | |
| Cytokine/Chemokine | | | Estimates | p value | Estimates | p value | Estimates | p value |
| logHu IL-1b | pg/ml | | 0.094 | 0.681 | −0.604 | 0.035 | −0.047 | 0.860 |
| logHu IL-1ra | pg/ml | | −0.178 | 0.269 | 0.041 | 0.850 | 0.053 | 0.817 |
| logHu IL-2 | pg/ml | | −0.078 | 0.644 | −0.482 | 0.012 | 0.179 | 0.341 |
| logHu IL-4 | pg/ml | | 0.335 | 0.426 | −0.798 | 0.140 | −0.190 | 0.661 |
| logHu IL-5 | pg/ml | | −0.131 | 0.606 | −0.832 | 0.025 | 0.119 | 0.724 |
| logHu IL-6 | pg/ml | | 0.286 | 0.156 | −0.301 | 0.216 | 0.095 | 0.712 |
| logHu IL-7 | pg/ml | | 0.026 | 0.933 | −0.617 | 0.119 | 0.199 | 0.550 |
| logHu IL-8 | pg/ml | | 0.568 | 0.319 | 0.168 | 0.721 | −0.175 | 0.756 |
| logHu IL-9 | pg/ml | | −0.174 | 0.291 | −0.190 | 0.330 | 0.545 | 0.011 |
| logHu IL-10 | pg/ml | | −0.232 | 0.298 | −0.395 | 0.163 | 0.351 | 0.217 |
| logHu IL-12 | pg/ml | | −0.202 | 0.438 | −0.529 | 0.115 | 0.413 | 0.177 |
| logHu IL-13 | pg/ml | | −0.100 | 0.699 | −0.533 | 0.096 | 0.467 | 0.236 |
| logHu IL-15 | pg/ml | | −0.053 | 0.660 | −0.325 | 0.019 | 0.092 | 0.557 |
| logHu IL-17 | pg/ml | | −0.578 | 0.158 | −0.619 | 0.262 | −0.578 | 0.556 |
| logHu Eotaxin | pg/ml | | −0.363 | 0.124 | −0.360 | 0.291 | 0.056 | 0.868 |
| logHu FGF basic | pg/ml | | −0.168 | 0.546 | −0.688 | 0.085 | 0.493 | 0.281 |
| logHu G-CSF | pg/ml | | −0.321 | 0.380 | −0.978 | 0.120 | −0.032 | 0.943 |
| logHu GM-CSF | pg/ml | | −0.036 | 0.839 | −0.589 | 0.001 | 0.191 | 0.368 |
| logHu IFN-g | pg/ml | | 0.038 | 0.879 | −0.708 | 0.024 | 0.015 | 0.960 |
| logHu IP-10 | pg/ml | | −0.048 | 0.877 | 0.241 | 0.568 | 0.104 | 0.818 |
| logHu MCP-1 | pg/ml | | 0.144 | 0.623 | −0.113 | 0.739 | 0.009 | 0.981 |
| logHu MIP-1a | pg/ml | | 0.196 | 0.591 | −0.388 | 0.301 | 0.051 | 0.890 |
| logHu PDGF-bb | pg/ml | | 0.097 | 0.798 | −0.165 | 0.720 | 0.794 | 0.301 |
| logHu MIP-1b | pg/ml | | 0.351 | 0.477 | −0.284 | 0.573 | −0.396 | 0.281 |
| logHu RANTES | pg/ml | | 0.249 | 0.444 | −0.452 | 0.224 | 0.382 | 0.631 |
| logHu TNF-a | pg/ml | | −0.033 | 0.865 | −0.646 | 0.012 | 0.035 | 0.875 |
| logHu VEGF | pg/ml | | 0.400 | 0.139 | −0.042 | 0.902 | 0.573 | 0.132 |
| sgp130 | μ g/ml | | −3.785 | 0.046 | −7.801 | 0.001 | −3.005 | 0.207 |
| logHu-sIL-6R | pg/ml | | −0.866 | 0.187 | −1.246 | 0.075 | −0.754 | 0.336 |
| logHu-sTNFRI | pg/ml | | −1.028 | 0.039 | 0.033 | 0.955 | 0.094 | 0.902 |
| logHu-sTNFRII | pg/ml | | −0.179 | 0.766 | 0.078 | 0.728 | 0.690 | 0.115 |
| DAS-28 0w | | | 0.306 | 0.000 | 0.259 | 0.097 | 0.403 | 0.003 |
| MMP | | | 0.000 | 0.645 | 0.000 | 0.464 | 0.002 | 0.023 |
| RF | | | 0.001 | 0.378 | 0.000 | 0.818 | 0.000 | 0.504 |
| VAS | | | 0.003 | 0.842 | 0.004 | 0.560 | 0.006 | 0.334 |
| Swollen joint count | | | 0.069 | 0.000 | 0.066 | 0.183 | 0.081 | 0.022 |
| Tender joint count | | | 0.067 | 0.000 | 0.074 | 0.166 | 0.042 | 0.193 |
| Stage | | | 0.092 | 0.418 | 0.393 | 0.162 | −0.197 | 0.232 |
| Class | | | 0.188 | 0.483 | 0.130 | 0.680 | 0.453 | 0.096 |

Multiple linear regression analysis was performed to find the correlation between DAS-28 value after 16 weeks of therapy and cytokine/chemokine/soluble receptor concentration. As a result thereof, it was found by phased multiple regression analysis that a combination of sgp130, logIL-8, logEotaxin, logIP-10, logTNFRI, logTNFRII, logIL-6, and logIL-VEGF is significantly correlated with a DAS-28 value after 16 weeks of therapy in naive patients who received tocilizumab therapy as shown in Table 6. Further, it was found that there is a very significant correlation even without using logIL-VEGF (Table 7).

Further, it was found that a combination of sgp130, logIP-10, and logGM-CSF is significantly correlated with a DAS-28 value after 16 weeks of therapy in switch patients who received tocilizumab therapy (Table 8).

Meanwhile, a combination of DAS-28 value prior to therapy, logIL-6 and logIL-13 was also found to be significantly correlated with the level of improvement in DAS-28 value for naive patients who received etanercept therapy (Table 9). Further, a combination of logIL-9, logTNF-$\alpha$, and logVEGF, even without using a DAS-28 value prior to therapy, is significantly correlated with a DAS-28 value after 16 weeks of therapy naive patients who received etanercept therapy (Table 10).

[Table 6]

Tocilizumab naïve multiple linear regression analysis
Objective variable 16-week DAS-28

Multiple linear regression anlysis of cytokine /chemokine
/soluble receptor based on 16w DAS-28
A.Multiple regression anlysis were performed to find the
parameters related to 16wDAS-28 (=16wDAS28).

| Naïve Tocilizumab Therapy | Tocilizumab naïve | |
|---|---|---|
| Multiple regression analysis (Objective value=16wDAS28) | | |
| R^2 | 0.646 | |
| ANOVA(Analysis of variance) | p<0.0001 | |
| Cytokine/Chemokine/soluble receptor | Estimate | p value |
| intercept | 6.909 | 0.001 |
| sgp130# | −0.534 | 0.002 |
| log IL-8 | 3.940 | <.0001 |
| log Eotaxin | −1.039 | <.0001 |
| log IP-10 | −1.002 | 0.002 |
| log sTNFRI | −2.580 | <.0001 |
| log sTNFRII | 1.407 | 0.030 |
| log IL-6 | 0.744 | 0.002 |
| log VEGF | −0.850 | 0.039 |

sgp130# : $\mu$ g/ml
others : pg/ml

[Table 7]

Tocilizumab naïve multiple linear regression analysis

Objective variable 16-week DAS-28

Multiple linear regression anlysis of cytokine /chemokine /soluble receptor based on 16w DAS-28

A.Multiple regression anlysis were performed to find the parameters related to 16wDAS-28 (=16wDAS28).

| Naïve Tocilizumab Therapy | Tocilizumab naïve | |
|---|---|---|
| Multiple regression analysis (Objective value=16wDAS28) | | |
| $R^2$ | 0.605 | |
| ANOVA(Analysis of variance) | $p<0.0001$ | |
| Cytokine/Chemokine/soluble receptor | Estimate | p value |
| intercept | 4.731 | 0.0127 |
| sgp130# | −0.543 | 0.003 |
| log IL−8 | 2.551 | <.0001 |
| log Eotaxin | −0.937 | 0.0004 |
| log IP−10 | −1.116 | 0.0007 |
| log sTNFRI | −2.010 | 0.0004 |
| log sTNFRII | 1.630 | 0.0152 |
| log IL−6* | 0.577 | 0.0096 |

sgp130# : μ g/ml

others : pg/ml

[Table 8]

Tocilizumab switch multiple linear regression analysis

Objective variable 16-week DAS-28

Multiple linear regression anlysis of cytokine /chemokine /soluble receptor based on 16w DAS-28

A.Multiple regression anlysis were performed to find the parameters related to 16wDAS-28 (=16wDAS28).

| Tocilizumab switch | | |
|---|---|---|
| Multiple regression analysis (Objective value=16wDAS28) | | |
| $R^2$ | 0.486 | |
| ANOVA(Analysis of variance) | $p<0.0001$ | |
| Cytokine/Chemokine/soluble receptor | Estimate | p value |
| intercept | 2.837 | 0.011 |
| sgp130# | −0.604 | 0.003 |
| log IP−10 | 0.714 | 0.003 |
| log GM-CSF | −0.622 | 0.0003 |

sgp130# : μ g/ml

others : pg/ml

[Table 9]

## Multiple linear regression analysis on naïve patients who received etanercept therapy

Multiple linear regression anlysis of cytokine/chemokine/soluble receptor and DAS28-CRP before therapy on 16week Das-28.
Objective variable: 16-week DAS-28

| Naïve Etanercept Therapy | | |
|---|---|---|
| Multiple regression analysis (Objective value=16wDAS28) | | |
| R^2 | 0.321 | |
| ANOVA(Analysis of variance) | p=0.0016 | |
| Cytokine/Chemokine/soluble receptor | estimate | p value |
| intercept | 0.081 | 0.907 |
| DAS28-CRP (Prior to therapy) | 0.522 | 0.000 |
| logHu IL-6 | −0.969 | 0.015 |
| log HuIL-13 | 1.409 | 0.015 |

[Table 10]

## Etanercept naïve multiple linear regression analysis
## Objective variable 16-week DAS-28

Multiple linear regression anlysis of cytokine /chemokine /soluble receptor based on 16w DAS-28
A.Multiple regression anlysis were performed to find the parameters related to 16wDAS-28 (=16wDAS28).

| Tocilizumab switch | | |
|---|---|---|
| Multiple regression analysis (Objective value=16wDAS28) | | |
| R^2 | 0.264 | |
| ANOVA(Analysis of variance) | p=0.0093 | |
| Cytokine/Chemokine/soluble receptor | Estimate | p value |
| intercept | 0.703 | 0.348 |
| log IL-9 | 0.646 | 0.007 |
| log TNF-α | −0.551 | 0.039 |
| log VEGF | 0.858 | 0.053 |

IL-9, TNF-α, VEGF : pg/ml

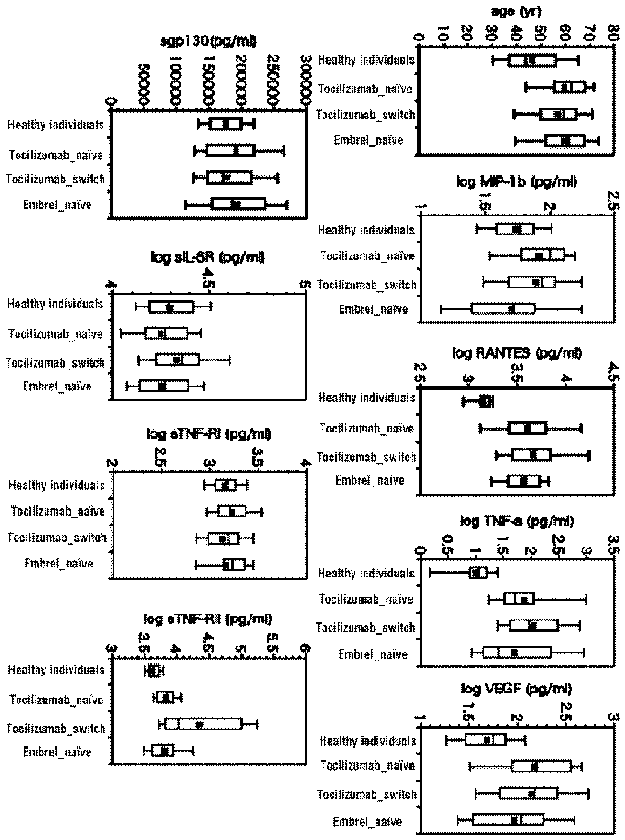

Further, regression equation (4) found based on the multiple linear regression analysis shown in Table 7 was used to find a predicted value of DAS-28 value after 16 weeks of therapy in naive patients who received tocilizumab therapy. Figure 5 shows the results of comparing predicted values of DAS-28 values after 16 weeks of therapy calculated by regression equation (4) and actual values of DAS-28 values after 16 weeks of therapy. It was confirmed from the results that DAS-28 values after 16 weeks of therapy estimated from the results of multiple linear regression analysis shown in Table 7 are very consistent with actual values of DAS-28 values after 16 weeks of therapy.

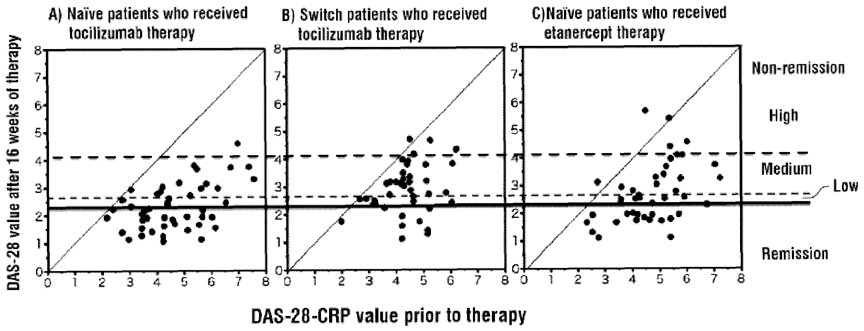

Further, regression equation (5) found based on the multiple linear regression analysis shown in Table 8 was used to find a predicted value of DAS-28 value after 16 weeks of therapy in switch patients who received tocilizumab therapy. Figure 6 shows the results of comparing the predicted values of DAS-28 values after 16 weeks of therapy calculated by regression equation (5) and actual values of DAS-28 values after 16 weeks of therapy. It was confirmed from the results that DAS-28 values after 16 weeks of therapy estimated from the results of multiple linear regression analysis shown in Table 8 are very consistent with actual values of DAS-28 values after 16 weeks of therapy.

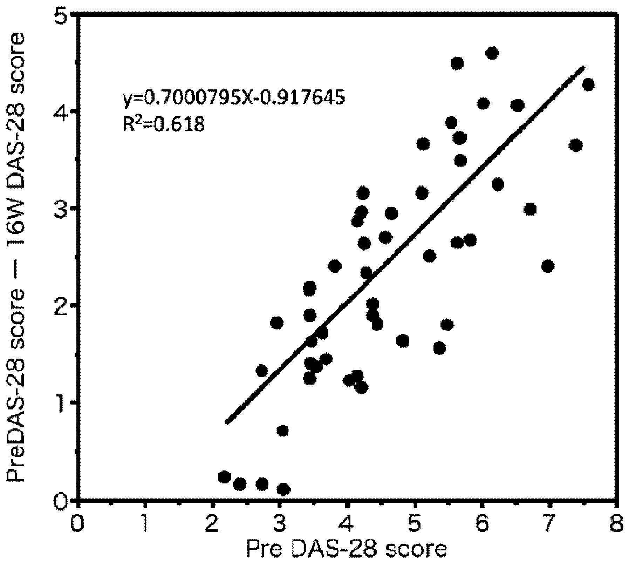

Regression equation (7) found based on the multiple linear regression analysis shown in Table 10 was used to find a predicted value of DAS-28 value after 16 weeks of therapy in naive patients who received etanercept therapy. Figure 7 shows the results of comparing predicted values of DAS-28 values after 16 weeks of therapy calculated by regression equation (7) and actual values of DAS-28 values after 16 weeks of therapy. It was confirmed from the results that DAS-28 values after 16 weeks of therapy can be estimated to a certain extent from the results of multiple linear regression analysis shown in Table 10.

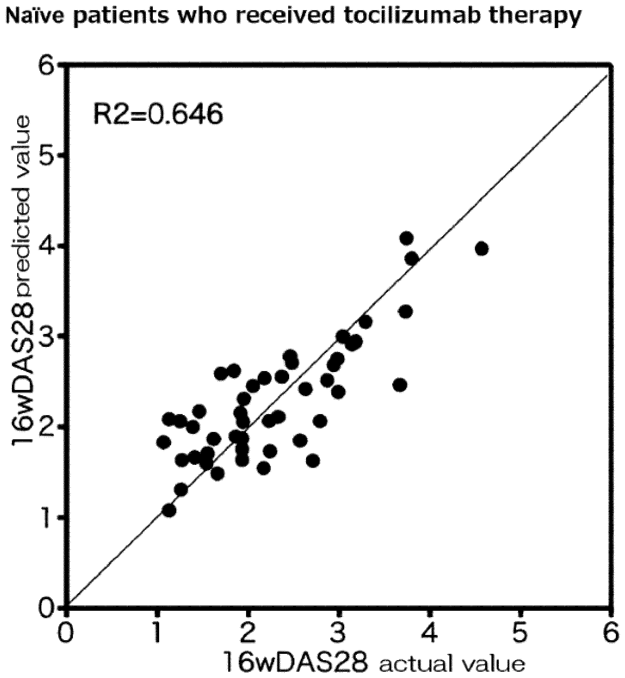

Further, a predicted value of DAS-28 after 16 weeks of therapy was found by using the aforementioned regression equation (4) while assuming that naive patients who received etanercept therapy had received tocilizumab therapy without receiving etanercept therapy. Figure 8 shows the actual values of DAS-28 after 16 weeks of etanercept therapy and predicted values of DAS-28 values after 16 weeks of therapy while assuming that tocilizumab therapy was received. From this result, naive patients who received etanercept therapy are classified into patients who are predicted to have a higher therapeutic effect when receiving tocilizumab therapy (Figure **8 a**), patients who are predicted to have barely any difference observed between etanercept therapy and tocilizumab therapy (Figure **8b**), and patients who are predicted to have a higher therapeutic effect observed when receiving etanercept therapy (Figure **8 c**). For patients shown in Figure **8 a**, tocilizumab therapy is estimated to be more effective than etanercept therapy that was actually received. Thus, it was found that a more effective therapeutic agent can be selected by estimating DAS-28 values due to tocilizumab therapy and etanercept therapy prior to therapy by the present invention.

(Search for biomarkers for predicting and determining the possibility of remission by using serum concentration of cytokines/chemokines/soluble receptors in rheumatism patient prior to therapy)

In therapy of rheumatoid arthritis, it is desirable that even a partial improvement is observed in the symptom of a patient. However, it is most desirable to reach complete remission. In this regard, in addition to a search for various factors for estimating the final DAS-28 value, a search was conducted for cytokines/chemokines/soluble receptors for predicting whether a patient reaches complete remission.

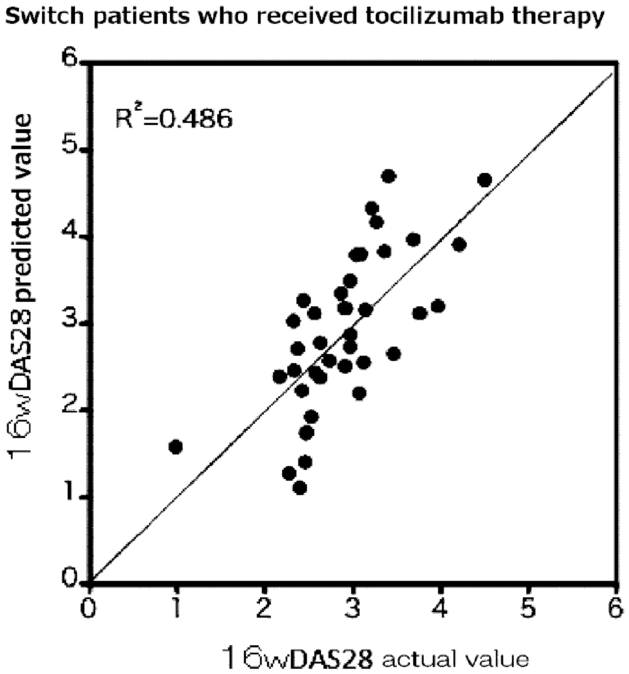

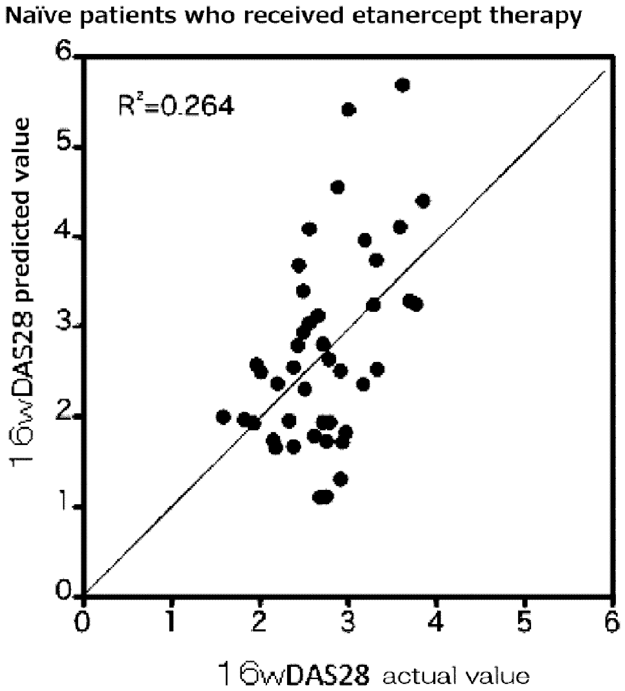

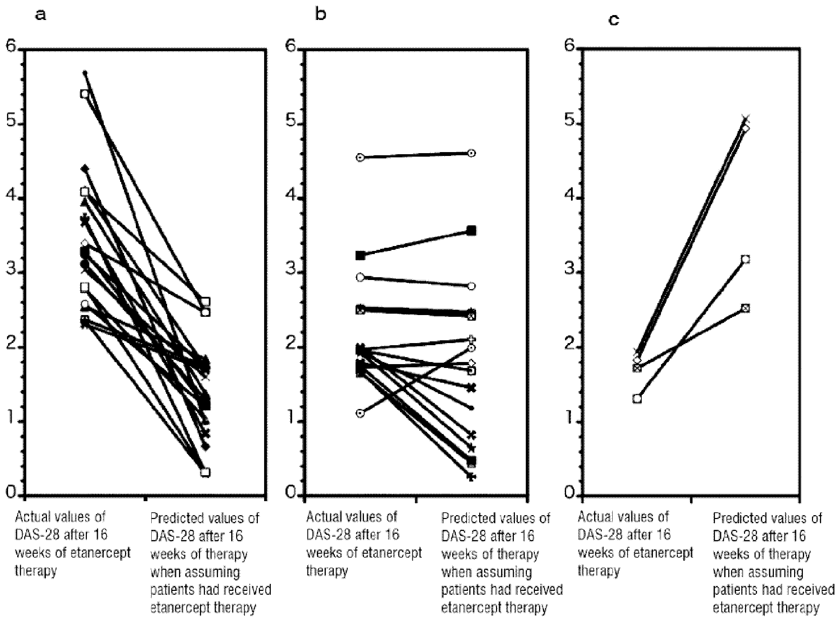

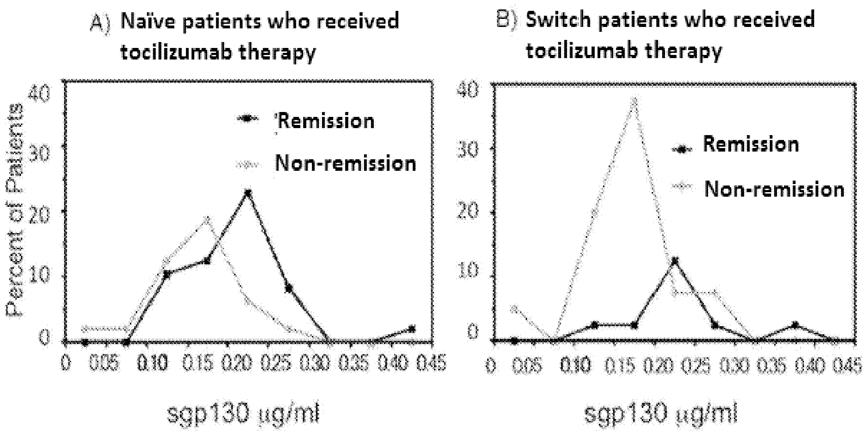

Data for cytokine/chemokine/soluble receptor concentrations was analyzed for complete remission and non-remission patient groups by simple logistic regression analysis. Further, Table 10 shows the results of analyzing data for cytokine/chemokine/soluble receptor concentrations for naive patients and switch patients who received tocilizumab therapy and naive patients who received etanercept therapy. It was found by simple logistic regression analysis that swollen joint count and tender joint count and DAS-28 values were significantly different between complete remission and non-remission groups. Furthermore, sgp130 was significantly different between complete remission and non-remission groups in naive and switch patients who received tocilizumab therapy (Table 11) . Meanwhile, significant difference in sgp130 was not observed between remission and non-remission groups in naive patients who received etanercept therapy (Table 11). Further, Figure 9 shows the results of analyzing the relationship between serum sgp130 concentration and DAS-28 value prior to therapy for remission and non-remission patients. As is clear from Figure **9,** many patients who have reached remission had a high sgpl30 concentration.

[Table 11]

## Simple logistic regression analysis

| Cytokine/Chemokine | | Naïve patients who received tocilizumab therapy (n=48) | | Switch patients who received tocilizumab therapy (n=40) | | Naïve patients who received etanercept therapy (n=43) | |
|---|---|---|---|---|---|---|---|
| | | Whole Model Test | Parameter Estimates | Whole Model Test | Parameter Estimates | Whole Model Test | Parameter Estimates |
| | | Single logistic analysis, p value | Estimates | Single logistic analysis, p value | Estimates | Single logistic analysis, p value | Estimates |
| logHu IL-1b | pg/ml | 0.378 | 0.486 | 0.147 | −1.081 | 0.577 | −0.274 |
| logHu IL-1ra | pg/ml | 0.148 | −0.628 | 0.087 | 1.573 | 0.323 | 0.478 |
| logHu IL-2 | pg/ml | 0.856 | 0.074 | 0.080 | −1.009 | 0.857 | 0.064 |
| logHu IL-4 | pg/ml | 0.534 | 0.638 | 0.420 | −1.241 | 0.965 | −0.035 |
| logHu IL-5 | pg/ml | 0.814 | 0.143 | 0.189 | −1.276 | 0.896 | −0.083 |
| logHu IL-6 | pg/ml | 0.184 | 0.663 | 0.270 | −0.710 | 0.950 | −0.030 |
| logHu IL-7 | pg/ml | 0.585 | 0.401 | 0.233 | −1.231 | 0.660 | 0.280 |
| logHu IL-8 | pg/ml | 0.432 | 1.088 | 0.864 | −0.207 | 0.751 | −0.333 |
| logHu IL-9 | pg/ml | 0.545 | −0.242 | 0.289 | −0.540 | 0.020 | 1.075 |
| logHu IL-10 | pg/ml | 0.604 | −0.281 | 0.196 | −0.948 | 0.572 | 0.310 |
| logHu IL-12 | pg/ml | 0.773 | −0.181 | 0.113 | −1.457 | 0.833 | 0.123 |
| logHu IL-13 | pg/ml | 0.963 | 0.029 | 0.432 | −0.669 | 0.671 | 0.322 |
| logHu IL-15 | pg/ml | 0.924 | 0.027 | 0.173 | −0.519 | 0.942 | 0.021 |
| logHu IL-17 | pg/ml | 0.197 | −1.332 | 0.920 | 0.147 | 0.691 | −0.730 |
| logHu Eotaxin | pg/ml | 0.447 | −0.441 | 0.512 | −0.590 | 0.639 | 0.299 |
| logHu FGF basic | pg/ml | 0.792 | −0.177 | 0.725 | −0.371 | 0.402 | 0.773 |
| logHu G-CSF | pg/ml | 0.599 | −0.471 | 0.786 | −0.450 | 0.901 | −0.104 |
| logHu GM-CSF | pg/ml | 0.910 | 0.104 | 0.099 | −0.930 | 0.798 | −0.102 |
| logHu IFN-g | pg/ml | 0.536 | 0.369 | 0.190 | −1.081 | 0.681 | −0.228 |
| logHu IP-10 | pg/ml | 0.647 | −0.344 | 0.604 | 0.557 | 0.393 | 0.733 |
| logHu MCP-1 | pg/ml | 0.402 | 0.593 | 0.696 | −0.366 | 0.960 | 0.035 |
| logHu MIP-1a | pg/ml | 0.428 | 0.698 | 0.305 | −0.963 | 0.885 | −0.098 |
| logHu PDGF-bb | pg/ml | 0.751 | 0.290 | 0.458 | 0.894 | 0.358 | 1.357 |
| logHu MIP-1b | pg/ml | 0.709 | 0.444 | 0.508 | −0.882 | 0.161 | −0.991 |
| logHu RANTES | pg/ml | 0.748 | 0.252 | 0.866 | 0.166 | 0.823 | −0.335 |
| logHu TNF-a | pg/ml | 0.787 | 0.127 | 0.143 | −1.020 | 0.694 | −0.162 |
| logHu VEGF | pg/ml | 0.400 | 0.558 | 0.389 | −0.793 | 0.967 | 0.030 |
| sgp130 | μ g/ml | −18.182 | 0.003 | −24.159 | 0.003 | 0.212 | −5.882 |
| logHu-sIL-6R | pg/ml | 0.118 | −2.590 | 0.023 | −5.922 | 0.679 | 0.590 |
| logHu-sTNFRI | pg/ml | 0.302 | −1.284 | 0.843 | −0.306 | 0.566 | 0.591 |
| logHu-sTNFRII | pg/ml | 0.719 | −0.519 | 0.064 | 1.210 | 0.390 | 0.775 |
| age | | 0.139 | 0.039 | 0.064 | −0.073 | 0.444 | 0.019 |
| Duretion of disease | | 0.228 | 0.041 | 0.221 | −0.059 | 0.414 | 0.033 |
| WBC | | 0.173 | 0.000 | 0.434 | 0.000 | 0.057 | 0.000 |
| DAS28-CRP | | 0.011 | 0.608 | 0.689 | 0.165 | 0.005 | 0.845 |
| VAS | | 0.328 | 0.013 | 0.810 | 0.005 | 0.419 | 0.011 |
| CRP | | 0.993 | 0.001 | 0.939 | −0.009 | 0.019 | 0.342 |
| RF | | 0.121 | 0.002 | 0.995 | 0.000 | 0.015 | 0.006 |
| Swollen joint count | | 0.015 | 0.123 | 0.193 | 0.182 | 0.012 | 0.218 |
| Tender joint count | | 0.014 | 0.123 | 0.363 | 0.137 | 0.046 | 0.147 |
| Stage | | 0.237 | 0.328 | 0.352 | 0.651 | 0.615 | −0.158 |
| Class | | 0.459 | 0.481 | 0.806 | −0.201 | 0.403 | 0.438 |

A multivariable model was examined as a prediction biomarker for remission and non-remission by phased multiple forward logistic regression analysis based on serum concentration of cytokines/chemokines/soluble receptors in patients prior to administration of tocilizumab. Tables 12 and 13 show optimal combinations of prediction biomarkers for remission and non-remission found based on phased multiple forward logistic regression analysis and ROC curves. It was found from the results of analysis that sgp130, logIP-10, logsTNFRII and logIL-6 can be prediction biomarkers for determining with high precision whether remission is reached for naive patients who received tocilizumab therapy (p = 0.0004) (Table 11a). Further, it was found that logIL-7 (p = 0.0003), logIL-1β (p = 0.0005) or logMCP-1 (p = 0.0004), in combination with sgp130, logIP-10, and logsTNFRII, can be a prediction biomarker for determining with high precision whether remission is reached for naive patients who received anti-IL-6 therapy (tocilizumab therapy) (Tables 12b-12d).

Further, it was found that a combination of sgp130, log IP-10, log sTNFRII and log IL-6 can be a prediction biomarker for determining whether remission is reached for switch patients who received tocilizumab therapy (p = 0.002) (Table 13a). Furthermore, it was also found that a combination of sgp130,log IP-10,log sTNFRII and log IL-1β can also be a predication biomarker for determining with high precision whether remission is reached (p = 0.003) (Table 13b).

Meanwhile, p value was 0.257 for biomarker groups for predicting and determining the possibility of remission found based on the ROC curve and multiple logistic regression analysis obtained in tocilizumab therapy for naive patients who received etanercept therapy, thus demonstrating that this biomarker group cannot predict whether remission is reached (Table 14) . Meanwhile, it was demonstrated that a combination of DAS-28 value prior to therapy (0wDAS-28), log VEGF, and log PDGF-bb can also predict and determine the possibility of remission to a certain extent, as shown in Table 15, by another multiple logistic regression analysis. Furthermore, it was found that a combination of log IL-9 and log TNF-α can also predict and determine the possibility of remission to a certain extent without using DAS-28 value (OwDAS-28) for naive patients who received etanercept therapy as shown in Table 16. That is, it is suggested that the pathology of rheumatoid arthritis patients is diverse, and a biomarker for predicting and determining the possibility of remission is different for patients to whom IL-6 inhibition is effective and patients for whom TNF-α inhibition is effective.

[Table 12]

# Tocilizumab naïve Multiple logistic regression analysis

## multiple logistic analysis, Objective variable:remission vs non-remission

| a. | Whole Model Test | p=0.0004 |
|---|---|---|
| Parameter Estimates | | |
| Term | Estimates | p value(Prob>ChiSq) |
| Intercept | -5,092 | 0,466 |
| sgp130 | -36,648 | 0,001 |
| logHu IP-10 | -4,004 | 0,007 |
| logHu-sTNFRII | 5,632 | 0,016 |
| logHu IL-6 | 1,658 | 0,034 |

Area Under
Curve=0.85009

[Table 13]

| b. | Whole Model Test | p=0.0003 |
|---|---|---|
| Parameter Estimates | | |
| Term | Estimates | p value(Prob>ChiSq) |
| Intercept | -3,467 | 0,621 |
| sgp130 | -42,849 | 0,001 |
| logHu IP-10 | -4,430 | 0,005 |
| logHu-sTNFRII | 5,736 | 0,017 |
| logHu IL-7 | 2,705 | 0,035 |

Area Under
Curve=0.84832

| c. | Whole Model Test | p=0.0004 |
|---|---|---|
| Parameter Estimates | | |
| Term | Estimates | p value(Prob>ChiSq) |
| Intercept | -2,834 | 0,684 |
| sgp130 | -38,721 | 0,001 |
| logHu IP-10 | -4,664 | 0,007 |
| logHu-sTNFRII | 5,369 | 0,020 |
| logHu MCP-1 | 2,502 | 0,040 |

Area Under
Curve=0.84832

[Table 13]

| d. | Whole Model Test | p=0.0005 |
|---|---|---|
| Parameter Estimates | | |
| Term | Estimates | p value(Prob>ChiSq) |
| Intercept | -1,269 | 0,853 |
| sgp130 | -39,538 | 0,001 |
| logHu IP-10 | -3,807 | 0,008 |
| logHu-sTNFRII | 5,086 | 0,026 |
| logHu IL-1b | 1,647 | 0,050 |

Area Under
Curve=0.85362

[Table 13]

# Tocilizumab switch Multiple logistic regression analysis
multiple logistic analysis, Objective variable: remission vs non-remission

| a. | Whole Model Test | p=0.0020 |
|---|---|---|
| Parameter Estimates | | |
| Term | Estimates | p value(Prob>ChiSq) |
| Intercept | -10,935 | 0,190 |
| sgp130# | -29,051 | 0,020 |
| logHu IP-10 | 4,466 | 0,060 |
| logHu-sTNFRII | 2,067 | 0,084 |
| logHu IL-6 | -2,757 | 0,047 |

Area Under Curve =0.892
sgp130#:μg/ml

[Table 14]

| b. | Whole Model Test | p=0.0030 |
|---|---|---|
| Parameter Estimates | | |
| Term | Estimate | Prob>ChiSq |
| Intercept | -9,671 | 0,217 |
| sgp130# | -27,150 | 0,026 |
| logHu IP-10 | 3,205 | 0,095 |
| logHu-sTNFRII | 1,914 | 0,090 |
| logHu IL-1b | -2,540 | 0,055 |

**Area Under Curve = 0.899**
**sgp130#:μg/ml**

[Table 14]

| Results of multiple logistic regression analysis on naive patients who received etanercept therapy by using biomarkers for predicting and determining the possibility of remission found based on results of multiple logistic regression analysis obtained from patients who received tocilizumab therapy | | |
|---|---|---|
| Whole Model Test | | p=0.257 |
| Parameter Estimates | | |
| Term | Estimates | p value(Prob>ChiSq) |
| Intercept | -6.489 | 0.179 |
| sgp130 | -9.591 | 0.150 |
| logHu IL-6 | -0.422 | 0.467 |
| logHu IP-1 0 | 0.893 | 0.435 |

(continued)

| Results of multiple logistic regression analysis on naive patients who received etanercept therapy by using biomarkers for predicting and determining the possibility of remission found based on results of multiple logistic regression analysis obtained from patients who received tocilizumab therapy | | |
|---|---|---|
| Whole Model Test | | p=0.257 |
| Parameter Estimates | | |
| Term | Estimates | p value(Prob>ChiSq) |
| logHu-sTNFR II | 1.789 | 0.235 |

[Table 15]

## Etanercept naïve Multiple logistic regression analysis multiple logistic analysis, Objective variable :remission vs non-remission

| Whole Model Test | | p=0.0034 |
|---|---|---|
| Parameter Estimates | | |
| Term | Estimates | p value(Prob>ChiSq) |
| Intercept | −19.058 | 0.025 |
| 0w DAS28−CRP | 1.390 | 0.007 |
| log VEGF | −2.763 | 0.045 |
| log PDGF−bb | 4.962 | 0.042 |

Area Under
Curve=0.8055

| Whole Model Test | | p=0.0071 |
|---|---|---|
| Parameter Estimates | | |
| Term | Estimates | p value(Prob>ChiSq) |
| Intercept | 4.491 | 0.031 |
| 0w DAS28−CRP | 1.107 | 0.007 |
| log MIP−1a | −1.808 | 0.082 |
| log PDGF−bb | 3.930 | 0.069 |

Area Under Curve=0.7986

[Table 16]

| Etanercept naive Multiple logistic regression analysis multiple logistic analysis, Objective variable:remission vs non-remission | | |
|---|---|---|
| Whole Model Test | | p=0.0115 |
| Parameter Estimates | | |
| Term | Estimates | p value(Prob>ChiSq) |
| Intercept | -1.004 | 0.337 |
| log IL-9 | 1.711 | 0.012 |
| log TNF-$\alpha$ | -1 .031 | 0.079 |

**Claims**

1. A method of predicting and determining a therapeutic effect of a biological formulation targeting an inflammatory cytokine on a rheumatoid arthritis patient, comprising the step of measuring a concentration of a determination marker in a serum collected from the rheumatoid arthritis patient prior to the administration of the biological formulation, wherein said determination marker is soluble glycoprotein 130 (sgp130) and said biological formulation is an anti-interleukin-6 (IL6) agent.

2. The method of claim 1, wherein the method is a method of predicting and determining a possibility of remission with tocilizumab.

3. The method of determining of claim 2, wherein a patient to be administered with tocilizumab is a rheumatoid arthritis patient who has not received anti-cytokine therapy in the past, and the determination marker is a combination of (i) sgp130, (ii) IP-10, (iii) sTNFRII, and (iv) IL-6, IL-7, MCP-1 or IL-1$\beta$.

4. The method of determining of claim 2, wherein a patient to be administered with tocilizumab is a rheumatoid arthritis patient who has received anti-cytokine therapy in the past, and the determination marker is a combination of (i) sgp130, (ii) IP-10, (iii) sTNFRII, and (iv) IL-6 or IL-1$\beta$.

5. The method of determining of claim 1, wherein the method is a method of predicting and determining a disease activity indicator after therapy with tocilizumab in a rheumatism patient who has not received anti-cytokine therapy in the past, and wherein the determination marker is a combination of sgp130, IL-8, Eotaxin, IP-10, sTNFRI, sTNFRII, and IL-6 or a combination of sgp130, IL-8, Eotaxin, IP-10, sTNFRI, sTNFRII, IL-6 and VEGF.

6. The method of determining of claim 1, wherein the method is a method of predicting and determining a value of a disease activity indicator after therapy with tocilizumab in a rheumatism patient who has received anti-cytokine therapy in the past, and the determination marker is a combination of sgp130, IP-10, and GM-CSF.

7. A method of selecting a more effective biological formulation for therapy in a rheumatism patient who has not received anti-cytokine therapy in the past from among biological formulations consisting of tocilizumab and etanercept, comprising:

   predicting and determining a possibility of remission with tocilizumab in accordance with the method of determining of claim 3;
   predicting and determining a possibility of remission with etanercept by measuring the concentration of determination markers in a serum collected from a rheumatism patient who has not received anti-cytokine therapy in the past, wherein the determination marker is a combination of IL-9 and TNF-$\alpha$, a combination of VEGF and PDGF-bb, or a combination of MIP-1$\alpha$ and PDGF-bb; and
   comparing the possibility of remission with tocilizumab with the possibility of remission with etanercept that were predicted and determined in the aforementioned steps to select a biological formulation with a high possibility of remission.

8. A method of selecting a more effective biological formulation for therapy in a rheumatism patient who has not received anti-cytokine therapy in the past from among biological formulations consisting of tocilizumab and etanercept, comprising:

predicting and determining a disease activity indicator after therapy with tocilizumab in accordance with the method of determining of claim 5;
predicting and determining a disease activity indicator after therapy with etanercept by measuring the concentration of determination markers in a serum collected from a rheumatism patient who has not received anti-cytokine therapy in the past, wherein the determination marker is a combination of IL-9, TNF-$\alpha$ and VEGF or a combination of IL-6 and IL-13; and
comparing the disease activity indicator after therapy with tocilizumab with the disease activity indicator after therapy with etanercept that were predicted and determined in the aforementioned steps to select a biological formulation with a low disease activity indicator after therapy.

9. Use of a diagnostic agent comprising a reagent capable of detecting a marker for predicting and determining a therapeutic effect due to a biological formulation targeting an inflammatory cytokine on a rheumatoid arthritis patient, wherein said determination marker is soluble glycoprotein 130 (sgp130) and said biological formulation is an anti-IL6 agent.

**Patentansprüche**

1. Verfahren zur Vorhersage und Bestimmung einer therapeutischen Wirkung einer biologischen Formulierung, die auf ein entzündliches Zytokin bei einem Patienten mit rheumatoider Arthritis abzielt, wobei das Verfahren den Schritt der Messung einer Konzentration eines Bestimmungsmarkers in einem Serum umfasst, welches dem Patienten mit rheumatoider Arthritis vor Verabreichung der biologischen Formulierung entnommen wurde, wobei der Bestimmungsmarker lösliches Glykoprotein 130 (sgp130) ist und die biologische Formulierung ein Anti-Interleukin-6 (IL6)-Mittel ist.

2. Verfahren nach Anspruch 1, wobei das Verfahren ein Verfahren zur Vorhersage und Bestimmung der Möglichkeit einer Remission mit Tocilizumab ist.

3. Verfahren zur Bestimmung nach Anspruch 2, wobei ein Patient, dem Tocilizumab zu verabreichen ist, ein Patient mit rheumatoider Arthritis ist, der in der Vergangenheit keine Anti-Zytokin-Therapie erhalten hat, und wobei der Bestimmungsmarker eine Kombination aus (i) sgpl30, (ii) IP-10, (iii) sTNFRII und (iv) IL-6, IL-7, MCP-1 oder IL-1$\beta$ ist.

4. Verfahren zur Bestimmung nach Anspruch 2, wobei ein Patient, dem Tocilizumab zu verabreichen ist, ein Patient mit rheumatoider Arthritis ist, der in der Vergangenheit eine Anti-Zytokin-Therapie erhalten hat, und wobei der Bestimmungsmarker eine Kombination aus (i) sgpl30, (ii) IP-10, (iii) sTNFRII und (iv) IL-6 oder IL-1$\beta$ ist.

5. Verfahren zur Bestimmung nach Anspruch 1, wobei das Verfahren ein Verfahren zur Vorhersage und Bestimmung eines Indikators für eine Krankheitsaktivität nach Therapie mit Tocilizumab bei einem Rheuma-Patienten ist, der in der Vergangenheit keine Anti-Zytokin-Therapie erhalten hat, und wobei der Bestimmungsmarker eine Kombination aus sgpl30, IL-8, Eotaxin, IP-10, sTNFRI, sTNFRII und IL-6 oder eine Kombination aus sgpl30, IL-8, Eotaxin, IP-10, sTNFRI, sTNFRII, IL-6 und VEGF ist.

6. Verfahren zur Bestimmung nach Anspruch 1, wobei das Verfahren ein Verfahren zur Vorhersage und Bestimmung eines Wertes eines Indikators für eine Krankheitsaktivität nach Therapie mit Tocilizumab bei einem Rheuma-Patienten ist, der in der Vergangenheit eine Anti-Zytokin-Therapie erhalten hat, und wobei der Bestimmungsmarker eine Kombination aus sgpl30, IP-10, und GM-CSF ist.

7. Verfahren zur Auswahl einer stärker wirksamen biologischen Formulierung für die Therapie bei einem Rheuma-Patienten, der in der Vergangenheit keine Anti-Zytokin-Therapie erhalten hat, aus einer Gruppe biologischer Formulierungen bestehend aus Tocilizumab und Etanercept, bei dem man:

die Möglichkeit einer Remission mit Tocilizumab nach dem Verfahren zur Bestimmung nach Anspruch 3 vorhersagt und bestimmt;
die Möglichkeit einer Remission mit Etanercept durch Konzentrationsmessung von Bestimmungsmarkern in einem Serum, welches einem Rheuma-Patienten entnommen wurde, der in der Vergangenheit keine Anti-Zytokin-Therapie erhalten hat, vorhersagt und bestimmt, wobei der Bestimmungsmarker eine Kombination aus IL-9 und TNF-$\alpha$, eine Kombination aus VEGF und PDGF-bb, oder eine Kombination aus MIP-l$\alpha$ und PDGF-bb ist; und

die Möglichkeit einer Remission mit Tocilizumab mit der Möglichkeit einer Remission mit Etanercept, die in den oben genannten Schritten vorhergesagt und bestimmt wurden, vergleicht, um eine biologische Formulierung mit einer großen Möglichkeit einer Remission auszuwählen.

8. Verfahren zur Auswahl einer stärker wirksamen biologischen Formulierung für die Therapie bei einem Rheuma-Patienten, der in der Vergangenheit keine Anti-Zytokin-Therapie erhalten hat, aus einer Gruppe biologischer Formulierungen bestehend aus Tocilizumab und Etanercept, bei dem man:

einen Indikator für eine Krankheitsaktivität nach Therapie mit Tocilizumab nach dem Verfahren zur Bestimmung nach Anspruch 5 vorhersagt und bestimmt;
einen Indikator für eine Krankheitsaktivität nach Therapie mit Etanercept durch Konzentrationsmessung von Bestimmungsmarkern in einem Serum, welches einem Rheuma-Patienten entnommen wurde, der in der Vergangenheit keine Anti-Zytokin-Therapie erhalten hat, vorhersagt und bestimmt, wobei der Bestimmungsmarker eine Kombination aus IL-9, TNF-$\alpha$ und VEGF oder eine Kombination aus IL-6 und IL-13 ist; und
den Indikator für eine Krankheitsaktivität nach Therapie mit Tocilizumab mit dem Indikator für eine Krankheitsaktivität nach Therapie mit Etanercept, die in den oben genannten Schritten vorhergesagt und bestimmt wurden, vergleicht, um eine biologische Formulierung mit einem niedrigen Indikator für eine Krankheitsaktivität nach der Therapie auszuwählen.

9. Verwendung eines diagnostischen Mittels, das ein Reagenz umfasst, welches in der Lage ist, einen Marker zur Vorhersage und Bestimmung einer therapeutischen Wirkung zu erkennen, die auf einer biologischen Formulierung beruht, welche auf ein entzündliches Zytokin bei einem Patienten mit rheumatoider Arthritis abzielt, wobei der Bestimmungsmarker lösliches Glykoprotein 130 (sgp130) und die biologische Formulierung ein Anti-IL6-Mittel ist.

## Revendications

1. Procédé visant à prédire et déterminer l'effet thérapeutique, sur un patient souffrant de polyarthrite rhumatoïde, d'une formulation biologique dont la cible est une cytokine inflammatoire, lequel procédé comporte une étape où l'on mesure la concentration d'un marqueur de détermination dans du sérum prélevé chez le patient souffrant de polyarthrite rhumatoïde avant l'administration de la formulation biologique, et dans lequel procédé ledit marqueur de détermination est la sgp130 (glycoprotéine 130 soluble) et ladite formulation biologique est un agent anti-IL6 (interleukine 6).

2. Procédé conforme à la revendication 1, lequel procédé est un procédé visant à prédire et déterminer la possibilité d'obtenir une rémission grâce au tocilizumab.

3. Procédé de détermination conforme à la revendication 2, dans lequel le patient devant recevoir l'administration de tocilizumab est un patient souffrant de polyarthrite rhumatoïde qui n'a reçu auparavant aucun traitement anti-cytokine, et le marqueur de détermination est une combinaison

i) de sgp130,
ii) de IP-10,
iii) de sTNFR-II,
iv) et de IL-6, IL-7, MCP-1 ou IL-1$\beta$.

4. Procédé de détermination conforme à la revendication 2, dans lequel le patient devant recevoir l'administration de tocilizumab est un patient souffrant de polyarthrite rhumatoïde qui a reçu auparavant un traitement anti-cytokine, et le marqueur de détermination est une combinaison

i) de sgp130,
ii) de IP-10,
iii) de sTNFR-II,
iv) et de IL-6 ou IL-1$\beta$.

5. Procédé de détermination conforme à la revendication 1, lequel procédé est un procédé visant à prédire et déterminer un indicateur de l'activité de la maladie après un traitement au tocilizumab chez un patient souffrant de rhumatisme qui n'a reçu auparavant aucun traitement anti-cytokine, et dans lequel procédé le marqueur de détermination est une combinaison de sgp130, de IL-8, d'éotaxine, de IP-10, de sTNFR-I, de sTNFR-II et de IL-6, ou une combinaison de sgp130, de IL-8, d'éotaxine, de IP-10, de sTNFR-I, de sTNFR-II, de IL-6 et de VEGF.

**6.** Procédé de détermination conforme à la revendication 1, lequel procédé est un procédé visant à prédire et déterminer la valeur d'un indicateur de l'activité de la maladie après un traitement au tocilizumab chez un patient souffrant de rhumatisme qui a reçu auparavant un traitement anti-cytokine, et dans lequel procédé le marqueur de détermination est une combinaison de sgp130, de IP-10 et de GM-CSF.

**7.** Procédé de sélection, parmi les formulations biologiques comprenant du tocilizumab ou de l'étanercept, d'une formulation biologique offrant un plus grand effet thérapeutique chez un patient souffrant de rhumatisme qui n'a reçu auparavant aucun traitement anti-cytokine, lequel procédé comporte les étapes suivantes :

- prédire et déterminer la possibilité d'obtenir une rémission grâce au tocilizumab, en opérant selon un procédé de détermination conforme à la revendication 3,
- prédire et déterminer la possibilité d'obtenir une rémission grâce à l'étanercept, en mesurant la concentration de marqueurs de détermination dans du sérum prélevé chez le patient souffrant de rhumatisme qui n'a reçu auparavant aucun traitement anti-cytokine, étant entendu que le marqueur de détermination est une combinaison de IL-9 et de TNF-$\alpha$, une combinaison de VEGF et de PDGF-bb, ou une combinaison de MIP-1$\alpha$ et de PDGF-bb,
- et comparer la possibilité d'obtenir une rémission grâce au tocilizumab avec la possibilité d'obtenir une rémission grâce à l'étanercept, possibilités prédites et déterminées au cours des étapes mentionnées ci-dessus, afin de sélectionner une formulation biologique offrant de grandes possibilités de rémission.

**8.** Procédé de sélection, parmi les formulations biologiques comprenant du tocilizumab ou de l'étanercept, d'une formulation biologique offrant un plus grand effet thérapeutique chez un patient souffrant de rhumatisme qui n'a reçu auparavant aucun traitement anti-cytokine, lequel procédé comporte les étapes suivantes :

- prédire et déterminer un indicateur de l'activité de la maladie après un traitement au tocilizumab, en opérant selon un procédé de détermination conforme à la revendication 5,
- prédire et déterminer un indicateur de l'activité de la maladie après un traitement à l'étanercept, en mesurant la concentration de marqueurs de détermination dans du sérum prélevé chez le patient souffrant de rhumatisme qui n'a reçu auparavant aucun traitement anti-cytokine, étant entendu que ledit marqueur de détermination est une combinaison de IL-9, de TNF-$\alpha$ et de VEGF ou une combinaison de IL-6 et de IL-13,
- et comparer l'indicateur de l'activité de la maladie après une thérapie au tocilizumab avec l'indicateur de l'activité de la maladie après un traitement à l'étanercept, indicateurs prédits et déterminés au cours des étapes mentionnées ci-dessus, afin de sélectionner une formulation biologique donnant un faible indicateur de l'activité de la maladie après traitement.

**9.** Utilisation d'un agent de diagnostic comprenant un réactif capable de détecter un marqueur, afin de prédire et déterminer l'effet thérapeutique, sur un patient souffrant de polyarthrite rhumatoïde, d'une formulation biologique dont la cible est une cytokine inflammatoire, pour laquelle ledit marqueur de détermination est la sgp130 (glycoprotéine 130 soluble) et ladite formulation biologique est un agent anti-IL6.

A) Naïve patients who received tocilizumab therapy

B) Switch patients who received tocilizumab therapy

C) Naïve patients who received etanercept therapy

DAS-28 value after 16 weeks of therapy

DAS-28-CRP value prior to therapy

Non-remission

High

Medium

Low

Remission

## Naïve patients who received tocilizumab therapy

## Switch patients who received tocilizumab therapy

Naïve patients who received etanercept therapy

A) Naïve patients who received tocilizumab therapy

B) Switch patients who received tocilizumab therapy

**EP 3 088 897 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011128096 A
- JP 2011182780 A
- WO 201241332 A
- JP 2009225713 A
- JP 2010088432 A

**Non-patent literature cited in the description**

- **STEINBROCKER O et al.** Therapeutic criteria in rheumatoid arthritis. *JAMA,* 1949, vol. 140, 659
- **HOCHBERG MC et al.** *The American College of Rheumatology,* 1991
- *Arthritis and Rheumatism,* 1992, vol. 35, 498-502
- **PERS Y.M. et al.** *Rheumatology,* 19 September 2013
- **KOIKE T.** *J. Rheumatology,* November 2013
- **MARKENSON JA et al.** *J. Rheumatology,* July 2011, 1273-81
- **CURITIS JR et al.** *Ann RheumDis.,* 2012, vol. 71, 206-212
- **KOIKE T et al.** *J. Rheumatology,* October 2013, 1658-1668
- **CANNON GW et al.** *Clin Exp Rheumatol.,* November 2013

54